(19) Europäisches Patentamt
European Patent Office
Office européen des brevets

(11) EP 0 549 525 B1

(12) EUROPÄISCHE PATENTSCHRIFT

(45) Veröffentlichungstag und Bekanntmachung des Hinweises auf die Patenterteilung:
**23.07.1997 Patentblatt 1997/30**

(51) Int. Cl.[6]: **C07K 16/44**, C12P 21/08, C12N 5/20, G01N 33/577, C07C 311/51, C07C 311/58

(21) Anmeldenummer: **92810949.5**

(22) Anmeldetag: **03.12.1992**

(54) **Monoklonale Antikörper gegen Sulfonylharnstoffherbiziden und ihre Verwendung zum Nachweis**

Monoclonal antibodies against sulfonylurea herbicides and their use for detection

Anticorps monoclonaux contre des herbicides de sulfonyluréum et leur utilisation pour la détection

(84) Benannte Vertragsstaaten:
**AT BE CH DE DK ES FR GB GR IE IT LI LU MC NL PT SE**

(30) Priorität: **12.12.1991 CH 3652/91**

(43) Veröffentlichungstag der Anmeldung:
**30.06.1993 Patentblatt 1993/26**

(83) **Erklärung nach Regel 28(4) EPÜ (Sachverständigenlösung)**

(73) Patentinhaber: **Novartis AG**
**4058 Basel (CH)**

(72) Erfinder:
- **Schlaeppi, Jean-Marc, Dr.**
  **CH-4051 Basel (CH)**
- **Hüglin, Dietmar, Dr.**
  **W-7800 Freiburg (DE)**

(56) Entgegenhaltungen:
**EP-A- 0 120 814**
**EP-A- 0 463 998**
**WO-A-91/04697**

EP 0 549 525 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Rank Xerox (UK) Business Services
2.14.11/3.4

## Beschreibung

Gegenstand der vorliegenden Erfindung sind monoklonale Antikörper, die sich durch eine hohe Selektivität und Affinität gegenüber Herbiziden aus der Gruppe der Sulfonylharnstoffe, insbesondere aber gegenüber Sulfonylharnstoff-Herbiziden der Formel (A),

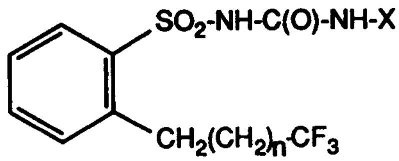

(A)

worin

X für einen über Kohlenstoff gebundenen, ein- oder zweifach substituierten 6-Ring-Heterozyklus mit ein bis 3 Stickstoffatomen, vorzugsweise aber für ein ein- bis zweifach substituiertes s-Triazin oder Pyrimidin steht; und
n eine ganze Zahl von 0 bis 4, vorzugsweise aber die Zahl 1 repräsentiert, auszeichnen und die sich daher in hervorragender Weise für die Verwendung in einem Immunassay zum schnellen und effektiven Nachweis von besagten Sulfonylharnstoff-Herbiziden in Boden-, Wasser- oder Luftproben oder auch in Pflanzenextrakten eignen sowie Verfahren zur Herstellung und Verwendung besagter monoklonaler Antikörper.

Insbesondere betrifft die vorliegende Erfindung monoklonale Antikörper, die sich durch eine hohe Selektivität und Affinität gegenüber Verbindungen der Formel (I) auszeichnen,

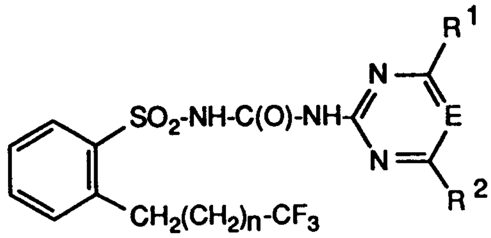

worin

$R^1$ und $R^2$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder -$NR^3R^4$, worin
$R^3$ und $R^4$ unabhängig voneinander für Wasserstoff oder $C_1$-$C_4$-Alkyl stehen;
E Stickstoff oder die Methinbrücke bedeuten; und
n eine ganze Zahl von 0 bis 4, vorzugsweise aber 1 repräsentiert.

In obiger Definitionen ist unter Alkyl geradkettiges oder verzweigtes Alkyl zu verstehen, wie z.B. Methyl, Aethyl, n-Propyl, i-Propyl sowie die vier isomeren Butyl. Bevorzugt sind Methyl und Aethyl.

Unter Alkoxy ist zu verstehen: Methoxy, Aethoxy, n-Propoxy, i-Propoxy, und die vier isomeren Butoxyreste, insbesondere aber Methoxy, Aethoxy und i-Propoxy.

Beispiele für Alkylthio sind Methylthio, Aethylthio, n-Propylthio, i-Propylthio sowie n-Butylthio, insbesondere aber Methylthio und Aethylthio.

Unter Halogen sind, unabhängig davon ob es einzeln oder als Bestandteil einer Halogenalkyl-, Halogenalkoxy-, oder Halogenaklylthio-Gruppe vorliegt, Fluor, Chlor und Brom, vorzugsweise aber Fluor und Chlor, zu verstehen. Beispiele für Halogenalkyl sind Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,-3,3-Hexafluorpropyl, insbesondere aber Fluormethyl, Chlormethyl, Difluormethyl und Trifluormethyl.

Besonders bevorzugt sind monoklonale Antikörper, die sich durch eine hohe Selektivität und Affinität gegenüber einer Verbindung der folgenden Formel (II) auszeichnen

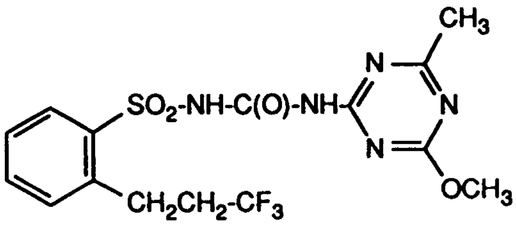

(II)

und die sich daher in hervorragender Weise für die Verwendung in einem Immunassay zum schnellen und effektiven Nachweis von besagtem Sulfonylharnstoff-Herbizid gemäss Formel (II) in Boden-, Wasser- oder Luftproben oder auch in Pflanzenextrakten eignen sowie Verfahren zur Herstellung besagter monoklonaler Antikörper.

Ein weiterer Aspekt der vorliegenden Erfindung betrifft Hybridomazellinien, die besagte monoklonale Antikörper produzieren sowie immunologische Verfahren zum Nachweis von Sulfonylharnstoff-Herbiziden der Formel (A) in Boden-, Wasser- oder Luftproben sowie in biologischem Material wie z.B. Pflanzenextrakten, unter Verwendung besagter monoklonaler Antikörper und die im Rahmen dieser Nachweisverfahren verwendbaren Testkits.

Bei den Sulfonylharnstoffen handelt es sich um eine neue Klasse besonders wirksamer Herbizide, die für die Zwecke des Pflanzenschutzes eingesetzt werden, insbesondere zur selektiven und effektiven Bekämpfung breitblättriger Unkräuter in Getreidekulturen.

Ein Vertreter aus dieser Klasse ist die oben genannte Verbindung der Formel (II), ein N-[2-(3,3,3-trifluorpropyl)-phenylsulfonyl]-N'-(4-methoxy-6-methyl-1,3,5-triazin-2-yl-)-harnstoff, der in erster Linie in Maiskulturen Anwendung findet zur selektiven Bekämpfung breitblättriger Unkräuter, wie z.B. *Amaranthus retroflexus* oder *Chenopodium album.*

Der Nachweis von Herbiziden aus der Gruppe der Sulfonylharnstoffe in Boden- und Wasserproben wird heute in erster Linie mittels Gas- oder Flüssigchromatographie (z.B. HPLC) durchgeführt [GC: Ahmad und Crawford (1990); HPLC: Zahnow (1982); Iwanzik und Egli (1988)]; van Rensburg E (1985)] sowie unter Verwendung von Bioassays.

Da z.T. bereits geringste Rückstände dieser Herbizide im Boden immer noch eine hohe Restaktivität aufweisen, die insbesondere für Sulfonylharstoff-empfindliche Folgekulturen schädlich sein können, ist es insbesondere für den Landwirt von besonderer Wichtigkeit ein zuverlässiges und in höchstem Masse sensitives Nachweisverfahren für die Rückstandsanalyse zur Verfügung zu haben.

Die meisten der zuvor genannten Verfahren zum Nachweis synthetischer Herbizide weisen zwar die für eine Rückstandsanalyse notwendige Sensitivität auf, deren Anwendung ist aber in der Regel noch mit zahlreichen Nachteilen verbunden. So müssen beispielsweise für die Bestimmung von Sulfonylharnstoff-Herbiziden in Bodenproben mittels GC oder HPLC vor der Durchführung der eigentlichen chromatographischen Analyse aufwendige Reinigungs- und Aufkonzentrierungsschritte zwischengeschaltet werden. Diese Verfahren sind somit nicht unter Feldbedingungen durchführbar, da sie an einen erheblichen Apparativen Aufwand gebunden sind.

Weitere Nachteile dieser Verfahren sind darin zu sehen, dass beispielsweise in der Gaschromatographie nur elementspezifische Detektoren eingesetzt werden, während bei der HPLC photometrische Detektoren verwendet werden, die relativ unspezifisch sind. Mit Ausnahme der massenspektroskopischen Detektion beruhen die chromatographischen Analysen auf der Bestimmung von Retentionszeiten der jeweiligen Substanz Diese Werte sind aber relativ und damit nicht strukturspezifisch.

Die zuvor ebenfalls erwähnten Bioassay-Verfahren sind zwar ohne grossen Aufwand durchführbar, zeigen aber eine zu geringe Spezifität.

Um diese zuvor beschriebenen Nachteile der etablierten analytischen Verfahren zu vermeiden hat man in jüngster Zeit versucht auch für den Agrarsektor immunologische Verfahren zu entwickeln, - wie sie in der klinischen Diagnostik zum Nachweis der verschiedensten Antigene bereits routinemässig eingesetzt werden - , insbesondere für die quantitative und qualitative Bestimmung von Agrarchemikalien in Boden-, Wasser- oder Luftproben.

So hat man beispielsweise mittlerweile begonnen, immunologische Verfahren für den Nachweis bestimmter Herbizide wie 2,4-Dichlorphenoxyessigsäure (Fleeker, 1986) oder Chlorsulforon (Kelley *et al*, 1985) sowie verschiedener Pestizide wie Diflubenzuron (Wie und Hammock, 1982), Metalaxyl (Newsome, 1985), Alachlor (Feng *et al*, 1990) oder Parathion (Ercegovich *et al*, 1981) zu entwickeln.

Auch für den immunologischen Nachweis von Herbiziden aus der Gruppe der Sulfonylharnstoffe sind somit bereits Verfahren beschrieben [Kelly et al. (1985); WO 91/04967]. WO 91/04967 beschreibt die Herstellung von polyklonalen Antikörpern mit Hilfe von Sulfonylharnstoffcarbonsäuren und deren Proteinkonjugaten.

Diese immunologischen Nachweisverfahren wie auch die zuvorgenannten Methoden beruhen auf der Verwendung polyklonaler Antiseren, die aus Tieren gewonnen werden, welche zuvor mit einem entsprechenden Antigen immunisiert wurden.

Die Herstellung monoklonaler Antikörper gegen Sulfonylharnstoff-Herbizide, die eine ausreichend hohe Affinität gegenüber der Zielsubstanz aufweisen und damit geeignet sind für den erfindungsgemässen Einsatz in einem der

bekannten Immunoassays, ist dagegen bisher nicht gelungen.

Polyklonale Antiseren sind in ihrer Zusammensetzung sehr heterogen, d.h. sie enthalten eine Vielzahl verschiedener Antikörper, die mit unterschiedlichen Epitopen des jeweiligen Antigens reagieren. Diese heterogene Zusammensetzung polyklonaler Antiseren kommt dadurch zustande, dass bei der Immunisierung eines Versuchstieres mit einem bestimmten Antigen immer mehrere Antikörper-produzierende Zellklone gleichzeitig stimuliert werden, von denen jeder ein anderes Epitop auf dem Antigenmolekül erkennt und daher von den stimulierten Zellklonen unterschiedliche Antikörper produziert werden.

Aus diesem Grund sind Seren immunisierter Tiere immer polyklonal und somit heterogen, sowohl was ihre Spezifität und als auch ihre Zugehörigkeit zu den einzelnen Klassen von Immunglobulinen angeht.

Diese Heterogenität in der Zusammensetzung polyklonaler Antiseren kann daher dazu führen, dass aufgrund zu geringer Affinität die Nachweisempfindlichkeit nicht ausreichend ist für einen selektiven Nachweis einer bestimmten Zielsubstanz oder dass strukturell nahe verwandte Verbindungen, wie beispielsweise die Zielsubstanz [Verbindung der Formel (II)] und deren Hydroxylierungsprodukte, bei der Verwendung polyklonaler Antikörper im Rahmen eines Immunassays nicht in ausreichendem Masse differenziert werden können.

Um diese Nachteile der polyklonalen Antiseren zu überwinden, wurden in jüngster Zeit vermehrt Anstrengungen unternommen auch für den Agrarsektor monoklonale Antikörper zu entwickeln. So berichten Schlaeppi *et al* (1989) über die Herstellung und Verwendung monoklonaler Antiköprer gegen Atrazin und Hydroxyatrazin einem Enzym-gekoppelten Immunassay.

Die Aufgabe, die es im Rahmen dieser Erfindung zu lösen galt, betraf somit in erster Linie die Bereitstellung eines einfach handhabbaren, effektiven und in hohem Masse selektiven Immunassays für einen schnellen und zuverlässigen Nachweis von Sulfonylharnstoff-Herbiziden der Formel (A), vorzugsweise aber zum Nachweis von Sulfonylharnstoff-Herbiziden der Formel (I) und ganz besonders bevorzugt zum Nachweis einer Verbindung der folgenden Formel (II),

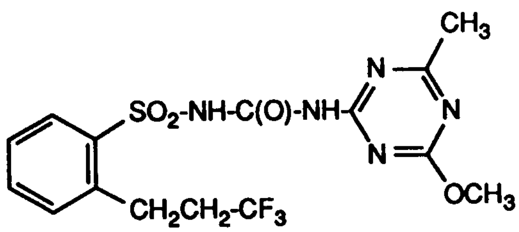

(II)

z.B. in Boden-, Wasser- und Luftproben.

Diese Aufgabe konnte jetzt im Rahmen der vorliegenden Erfindung überraschenderweise gelöst werden und zwar durch die Bereitstellung von monoklonalen Antikörpern mit hoher Spezifität und Affinität gegnüber Sulfonylharnstoff-Herbiziden der Formel (A), insbesondere aber gegenüber einer Verbindung der Formel (II), unter Verwendung der an sich bekannten Hybridoma/monoklonalen Antikörper-Technologie.

Die Verwendung hybrider somatischer Zellinien (Hybridomas) als Quelle für Antikörper gegen ganz bestimmte Antigene geht auf die Arbeiten von Köhler und Milstein (Nature, 256: 495-97, 1975) zurück.

Die Antikörper, die sich mit dem dort beschriebenen Verfahren gewinnen lassen, unterscheiden sich sehr stark von denjenigen, die man aus Antiseren konventionell immunisierter Tiere erhält.

Das Prinzip der Hybridoma/monoklonalen Antikörper-Technologie gründet sich auf die Beobachtung, dass beim Verschmelzen zweier somatischer Zellen die resultierende Hybridzelle charakteristische Merkmale beider Elterntypen aufweist.

Im Falle der monoklonalen Antikörperproduktion stammt die Fähigkeit zur Synthese des spezifischen Antikörpers von einer immunkompetenten B-Zelle (gewöhnlich einer Milzzelle), die einem zuvor immunisierten Donor-Tier entnommen wurde, während die Fähigkeit zur kontinuierlichen Zellteilung in Kultur durch den anderen Fusionspartner, eine Tumorzellinie (oft ein Myeloma) beigesteuert wird.

Für die Immunisierung der Donortiere werden in der Regel Konjugate bestehend aus dem Zielmolekül und einem damit verknüpften hochmolekularen Carriermolekül verwendet.

Jede dieser Hybrid-Zellinien synthetisiert ein homogenes Immunglobulin, das nur einen einzigen Vertreter aus der grossen Anzahl möglicher Antikörper repräsentiert, die von einem Tier als Antwort auf ein Antigen *in vivo* synthetisiert werden kann.

Da jeder Immunglobulin-produzierende Klon durch einen einzigen Typ von Antikörpern charakterisiert wird, hat sich die Bezeichnung monoklonale Antikörper eingebürgert.

Die Vorteile monoklonaler gegenüber polyklonalen Antikörpern sind zahlreich:

a) monokolonale Antikörper können in grosser Anzahl und in hohem Reinheitsgrad erhalten werden,

b) die Herstellung monoklonaler Antikörper ist homogen im Hinblick auf die Antigen-Reaktivität und ändert sich

auch nicht im Verlaufe der Zeit,

c) monoklonale Antikörper produziernde Hybridomas können über Jahre und Jahrzehnte hinweg aufbewahrt werden, ohne dabei ihre spezifischen Eigenschaften, i.e. die Produktion spezifischer monoklonaler Antikörper, zu verlieren,

d) monoklonale Antikörper sind für die Verwendung als Standardreagentien besser geeignet als polyklonale Antiseren, da letztere durch eine Vielzahl variabler Faktoren negativ beeinflusst werden. Dazu gehört beispielsweise

α) die Blutentnahme immunisierter Tiere zur Gewinnung des Antiserums,
β) das Problem der ständigen Verfügbarkeit von Material für zusätzliche Immunisierungen,
γ) die begrenzte Lebenszeit der Donortiere.

Monoklonale Antikörper, die mittlerweile gegen eine Vielzahl von Antigenen hergestellt wurden, sind in erster Linie in der medizinischen Diagnostik gut etabliert und aus dieser nicht mehr wegzudenken.

Im Rahmen der vorliegenden Erfindung wird jetzt erstmals ein monokonaler Antikörper mit hoher Spezifität und Affinität gegenüber Sulfonylharnstoff-Herbiziden der Formel (A), insbesondere aber gegenüber einer Verbindung der Formel (II), zur Verfügung gestellt, der unter Anwendung der an sich bekannten und zuvor kurz skizzierten Hybridoma/monoklonalen AntikörperTechnologie herstellbar ist und der aufgrund seiner hohen Affinität gegenüber der Zielsubstanz für einen erfindungsgemässen Einsatz in einem der bekannten Immunoassays geeignet ist.

Bei der Herstellung monoklonaler Antikörper gegen Sulfonylharnstoff-Herbizide der Formel (A) geht man im einzelnen so vor, dass man bei der Herstellung des für die Immunisierung vorgesehenen Immunogens ein Fragment verwendet, das insbesondere den Sufonamidteil des Moleküls umfasst, und dieses Fragment mit einem der üblicherweise verwendeten, hochmolekularen Carriermoleküle verknüpft.

Nach Auslösen der Immunantwort im Donortier werden die für die Antikörperproduktion verantwortlichen Zellen in der nachfolgend im einzelnen beschriebenen Weise aus dem Donortier isoliert und zur Herstellung von Hybridomazellen mit geeigneten Myelomazellen fusioniert.

Gegenstand der vorliegenden Erfindung ist somit ein Verfahren zur Herstellung eines monoklonalen Antikörpers mit hoher Spezifität und Affinität gegenüber einem oder mehreren Sulfonylharnstoff-Herbiziden gemäss Formel (A), das sich dadurch kennzeichnet, dass man

(a) eine Kupplungskomponente, die aus einem Fragment besteht, das insbesondere den Sulfonamidteil der Verbindung der Formel A umfasst mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert.

Auch bei der Herstellung monoklonaler Antikörper gegen eine Verbindung der Formel (I), insbesondere aber bei der Herstellung monoklonaler Antikörper der Formel (II) wird auf die zuvor beschriebene Weise vorgegangen.

Die aus dem erfindungsgemässen Verfahren erhältlichen monoklonalen Antikörper bilden einen weiteren Gegenstand der vorliegenden Erfindung. Bevorzugt sind monoklonale Antikörper mit hoher Spezifität bzw Affinität gegenüber Sulfonylharnstoff-Herbiziden der Formel (A), vorzugsweise aber gegenüber Sulfonylharnstoff-Herbiziden der Formel (I) und ganz besonders bevorzugt gegenüber einer Verbindung der Formel (II), die durch ein Verfahren, das dadurch gekennzeichnet ist, dass man

(a) eine Kupplungskomponente, die aus einem Fragment besteht, das insbesondere den Sulfonamidteil des Zielmoleküls, umfaßt mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert, herstellbar sind.

Ganz besonders bevorzugt ist ein monoklonaler Antikörper, der aus der Hybridomazellinie mit den kennzeichnenden Charakteristika von ECACC 911 20619 erhältlich ist, sowie Derivate von besagtem monoklonalem Antikörper.

Unter Derivaten monoklonaler Antikörper sind im Rahmen der vorliegenden Erfindung z.B. Antikörperfragmente zu

verstehen, die noch die hohe Spezifität und Affinität für die antigenen Determinanten der Sulfonylharnstoff-Herbizide der Formel (A), insbesondere aber für die antigenen Determinanten einer Verbindung der Formel (II) besitzen, desweiteren radioaktiv markierte monoklonale Antikörper, die beispielsweise mit radioaktivem Jod ($^{125}I$, $^{131}I$) Kohlenstoff ($^{14}C$), Schwefel ($^{35}S$), Tritium ($^{3}H$) oder ähnlichem markiert sind, Konjugate monoklonaler Antikörper mit Biotin oder Avidin, mit Enzymen, wie Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase oder Glucose-6-phosphatdehydrogenase, desweiteren Konjugate monoklonaler Antikörper mit biolumineszierenden (z.B. Luciferase), chemolumineszierenden (z.B. Acridiniumester) oder fluoreszierenden (z.B. Phycobiliproteine) Agentien. Ebenso umfasst von der vorliegenden Anmeldung sind bispezifische sowie sog. "cross-linked" Antikörper. Diese beispielhafte Aufzählung möglicher Antikörperderivate dient lediglich der Illustration der vorliegenden Erfindung und soll den Erfindungsgegenstand in keiner Weise limitieren.

Unter der Bezeichnung "im wesentlichen keine Kreuzreaktivität" soll im Rahmen dieser Erfindung verstanden werden, dass die Reaktivität der für Sulfonylharnstoff-Herbizide der Formel (A), insbesondere aber für eine Verbindung der Formel (II) spezifischen monoklonalen Antikörper mit unspezifischen Epitopen anderer Verbindungen, insbesondere strukturell verwandter Verbindungen wie z.B. Triasulfuron, in der Mehrzahl der Fälle weniger als 10 %, vorzugsweise aber weniger als 1.5 % und ganz besonders bevorzugt weniger als 0.1 % beträgt.

Der prozentuale Anteil der Kreuzreaktivität soll im Rahmen dieser Erfindung definitionsgemäss durch die folgende Beziehung wiedergegeben werden:

[Antigenkonzentration für 50 % Hemmung ($I_{50}$)/Konzentration der Antigenanalogen für 50 % Hemmung ($I_{50}$)] x 100.

Der $I_{50}$-Wert kann beispielsweise mit Hilfe eines kompetitiven ELISA Assays (vgl. Beispiel 8) bestimmt werden. Diese entspricht dann beispielsweise derjenigen Antigenkonzentration, die zu einer 50 %igen Hemmung der Antikörperbindung an das Träger-gebundene Antigen führt.

Ein weiterer Gegenstand der vorliegenden Erfindung betrifft Hybridomazellinien, welche die zuvor näher charakterisierten monoklonalen Antikörper synthetisieren und vorzugsweise in das umgebende Medium sezernieren.

Insbesondere betrifft die vorliegende Erfindung eine Hybridomazellinie, die einen monoklonalen Antikörper produziert, der eine hohe Spezifität und Affinität gegenüber Sulfonylharnstoff-Herbiziden der Formel (A), vorzugsweise aber gegenüber Sulfonylharnstoff-Herbiziden der Formel (I) und ganz besonders bevorzugt gegenüber einer Verbindung der Formel (II) aufweist, und die einen monoklonalen Antikörper produziert, der eine hohe Spezifität und Affinität gegenüber einer oder mehreren Verbindungen der Formel (A),

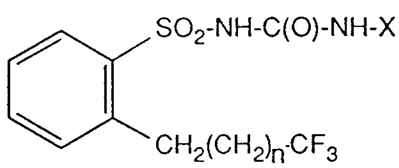

(A)

worin

X für einen über Kohlenstoff gebundenen, ein- oder zweifach substituierten 6-Ring-Heterozyklus mit ein bis 3 Stickstoffatomen steht; und

n eine ganze Zahl von 0 bis 4 repräsentiert, aufweist und herstellbar ist durch ein Verfahren, das dadurch gekennzeichnet ist, dass man

(a) eine Kupplungskomponente, die aus einem Fragment besteht, das insbesondere den Sulfonamidteil des Zielmoleküls umfaßt, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert; und
(e) das entstehende Fusionsprodukt isoliert und nach Selektion diejenigen Hybridomazellen kloniert, die den gewünschten Antikörper produzieren.

Ganz besonders bevorzugt ist eine Hybridomazellinie, die einen der erfindungsgemässen monoklonalen Antikörper produziert, und die die kennzeichnenden Charakteristika von ECACC 911 20619 besitzt, sowie Klone und Subklone davon.

Ebenso umfasst von der vorliegenden Erfindung sind Varianten und Mutanten der zuvor näher charakterisierten

Hybridomazellinien, die spontan entstehen oder aber artifiziell mit Hilfe bekannter Verfahren hergestellt werden können und die noch die charakteristischen Eigenschaften des Ausgangsmaterials aufweisen, d.h. die noch in der Lage sind die erfindungsgemässen Antikörper oder Derivate davon zu produzieren und diese vorzugsweise ins umgebende Medium zu sezernieren.

Ebenso umfasst von der vorliegenden Erfindung sind Verfahren zur Herstellung besagter Hybridomazellinien sowie Verfahren zur Herstellung besagter monoklonaler Antikörper.

Unter Klonen und Subklonen von Hybridomazellinien sind Hybridomas zu verstehen, die durch wiederholtes Klonieren aus dem Ausgangsklon hervorgehen und die noch die erfindungswesentlichen Merkmale des Ausgangsklons aufweisen.

Ein weiterer Gegenstand dieser Erfindung betrifft ein Verfahren zum immunologischen Nachweis von Sulfonylharnstoff-Herbiziden der Formel (A), vorzugsweise aber zum Nachweis von Sulfonylharnstoff-Herbiziden der Formel (I) und ganz besonders bevorzugt zum Nachweis einer Verbindung insbesondere der Formel (II), z.B. in Boden-, Wasser- oder Luftproben sowie in biologischem Material, wie z.B. in pflanzlichen oder tierischen Extrakten, unter Verwendung der erfindungsgemässen monoklonalen Antikörper.

Ebenso ein Bestandteil der vorliegenden Erfindung sind Mittel für die qualitative und quantitative Bestimmung von Sulfonylharnstoff-Herbiziden der Formel (A), vorzugsweise aber von Sulfonylharnstoff-Herbiziden der Formel (I) und ganz besonders bevorzugt zur Bestimmung einer Verbindung der Formel (II), in Form gebrauchsfertiger Test-Kits, welche mindestens einen der erfindungsgemässen monoklonalen Antikörper als Reagenz enthalten. Besonders bevorzugt ist ein Test-Kit, der für die Verwendung unter Feldebingungen zum schnellen und zuverlässigen Nachweis von den genannten Sulfonylharnstoff-Herbiziden geeignet ist.

Die Herstellung der erfindungsgemässen monoklonalen Antikörper erfolgt mit Hilfe an sich bekannter Verfahren, die im wesentlichen auf den von Köhler und Milstein (1975) entwickelten Methoden beruhen.

Da es sich bei den zu analysierenden Zielsubstanzen [Sulfonylharnstoff-Herbizide der Formel (A)] wie beispielsweise der Verbindung gemäss Formel (II), für die spezifische monoklonale Antikörper entwickelt werden sollen, um relativ kleine und einfache Moleküle handelt, die nach der Applikation in ein Versuchstier alleine nicht in der Lage sind dort eine entsprechende Immunantwort auszulösen, müssen vor der eigentlichen Immunisierung zunächst vorbereitende Massnahmen getroffen werden.

Man bezeichnet solche Verbindungen, die aufgrund ihrer Grösse und einfachen Strukturierung nicht zur Induktion einer Immunreaktion in der Lage sind, als Haptene oder inkomplette Antigene und stellt sie somit den kompletten Antigenen (= Immunogene) gegenüber, die sowohl als Antigen wirken als auch eine Immunantwort zu induzieren vermögen. Solche Haptenmoleküle können an hochmolekulare Verbindungen (Trägermoleküle) konjugiert werden, wodurch sie in ihren Eigenschaften kompletten Antigenen vergleichbar werden, d.h. sie besitzen jetzt die Fähigkeit zur Auslösung einer Immunantwort.

Einige der im Verlaufe der Immunisierungsreaktion gebildeten Antikörper sind dann in der Lage mit spezifischen Epitopen auf dem Haptenmolekül zu reagieren, unabhängig davon, ob das Haptenmolekül alleine oder aber nach wie vor gekoppelt an das Carriermolekül vorliegt.

Im Rahmen dieser Erfindung wird daher die als Hapten wirkende Zielsubstanz vor der Immunisierung von Versuchstieren an einen hochmolekularen Träger ('Carrier') gekoppelt, der geeignet ist dieser eine komplette antigene Wirksamkeit zu verleihen.

Unter geeigneten Carrier-Molekülen sind im Rahmen dieser Erfindung in erster Linie makromolekulare Verbindungen zu verstehen, die frei zugängliche reaktive Gruppen für die Kupplungsreaktion mit dem Hapten aufweisen und die in der Lage sind, durch Kupplung an das Hapten, diesem eine immunogene Potenz zu verleihen oder aber deren bereits vorhandene Immunogenizität zu verstärken.

Besonders bevorzugt im Rahmen dieser Erfindung sind makromolekulare Verbindungen, die frei zugängliche reaktive Aminogruppen enthalten.

Ganz besonders bevorzugt für die erfindungsgemässe Verwendung als Carriermolekül sind lysinreiche Proteine mit einem Molekulargewicht zwischen 10'000 und 1'500'000, wie z.B. "Bovine Serum Albumin" (BSA: MG 66'200), Humanes Serum Albumin (HSA,; MG 58'000) oder "Keyhole Limpet Hemocyanin" (KLH; MG > 1'000'000), die kommerziell erhältlich sind und somit in beliebiger Menge zur Verfügung stehen. Selbstverständlich können im Rahmen der vorliegenden Erfindung auch andere makromolekulare Verbindungen als Carrier-Moleküle verwendet werden, sofern sie die oben genannten Voraussetzungen erfüllen, wie z.B. "Porcine Thyroglobulin", B2 Microglobulin, Hemocyanin, Immunglobuline, Toxine (Cholera-, Tetanus-, Diphtheria-Toxin u.a.), Polysaccharide, Lipopolysaccharide, natürliche oder synthetische Polyadenyl-und Polyuridylsäuren, Polyalanyl- und Polylysin-Polypeptide oder Zellmembrankomponenten, wie beispielsweise Formalin- oder Glutaraldehyd-behandelte Erythrozytenzellmembranen.

Ebenso geeignet für die Verwendung als Carriermolekül in dem erfindungsgemässen Verfahren ist beispielsweise die gereinigte IgG Fraktion gegen Maus IgG (H+L) aus Kaninchen gemäss dem bei H Kawamura und JA Berzofsky (1986) beschriebenen Verfahren.

Die Konjugation des Haptens an das Trägermolekül kann entweder direkt oder aber vorzugsweise über ein Brükkenglied erfolgen, welches gegebenenfalls zunächst an das Haptenmolekül angelagert wird.

Die Kupplung der zu analysierenden Substanz an das Trägermolekül muss dabei in einer Weise erfolgen, dass die relevanten Strukturelemente der Zielsubstanzen frei zugänglich bleiben und somit in der Lage sind eine spezifische Immunantwort auszulösen, d.h. die Bildung spezifischer Antikörper zu induzieren. Bei der Herstellung von Sulfonylharnstoff-Derivaten, insbesondere aber von Derivaten der in Formel (II) wiedergegebenen Verbindung, ist daher darauf zu achten, dass diese Strukturelemente erhalten bleiben.

Dies lässt sich beispielsweise dadruch erreichen, dass man ein Sulfonylharnstoff-Herbizid der Formel (A), insbesondre aber eine Verbindung der Formel (II), in einer Weise derivatisiert, die eine Ankopplung an eine der zuvor genannten Carrier-Moleküle ohne Beeinträchtigung besagter relevanter Strukturelemente ermöglicht.

Besonders bevorzugt im Rahmen der Derivatisierung ist dabei die Verwendung ausgewählter Molekül-Fragmente anstelle des gesamten intakten Sulfonylharnstoffmoleküls. Auf diese Weise lassen sich somit monoklonale Antikörper mit hoher Affinität und Selektivität gegenüber Sulfonylharnstoff-Herbiziden der Formel (A), insbesondere aber gegenüber einer Verbindung der Formel (II), dadurch herstellen, dass man anstelle des komplexen Gesamtmoleküls, bestehend aus Sulfonamidteil, Harnstoffbrücke und Heterocyclusteil, ausschliesslich den Sulfonamidteil als Kupplungskomponente verwendet und diesen, wie zuvor beschrieben, mit einem geeigneten Carrielmolekül verknüpft. Dies ist von grossem verfahrenstechnischem Vorteil, da die Handhabung von Molekül-Fragmenten einfacher ist als diejenige des sehr viel komplexeren Gesamtmoleküls.

Wie im Rahmen der vorliegenden Erfindung gezeigt werden konnte, führt die Immunisierung von Donortieren mit dem zuvor beschriebenen Konjugat, bestehend aus einem der üblicherweise verwendeten, hochmolekularen Carrier und dem Sulfonamidteil eines Sulfonylharnstoff-Herbizids gemäss Formel (A), überräschenderweise zur Produktion hochspezifischer Antikörper.

Ein wesentlicher Aspekt der vorliegenden Erfindung besteht somit in der Verwendung des Sulfonamidteils von Sulfonylharnstoff-Herbiziden gemäss Formel (A), vorzugsweise aber von Sulfonylharnstoff-Herbiziden der Formel (I) und ganz besonders bevorzugt von einer Verbindung der Formel (II), zur Herstellung von Konjugaten, die für die Immunisierung von Donortieren eingesetzt werden können und letztlich zur Produktion monoklonaler Antikörper mit hoher Affinität gegenüber besagten Herbizidmolekülen führen.

Bei besagtem Sulfonamidteil handelt es sich vorzugsweise um substituiertes Arylsulfonyl, wobei der Arylrest vorzugsweise ein Phenyl ist. Typische Vertreter von unter den Umfang der Formel (A) fallenden Sulfonylharnstoff-Herbiziden sind z.B. in EP-0,120,814 beschrieben.

In der vorliegenden Erfindung wird daher nicht das gesamte Sulfonylharnstoff-Molekül an das Trägermolekül gekoppelt, sondern nur ein ausgewählter Teil davon, der die jeweils gewünschte determinante Gruppe in einer Form enthält, die in der Lage ist, eine sehr spezifische Immunantwort auszulösen. Besonders bevorzugt im Rahmen dieser Erfindung ist ein Fragment der in Formel (II) wiedergegebenen Verbindung, das auf den Sulfonamidanteil beschränkt bleibt.

Besagter Sulfonamidanteil kann dabei beispielsweise dadurch erhalten werden, dass man das entsprechende Sulfonylharnstoff-Herbizid an der Sulfonamid-Funktion nach an sich bekannten Methoden hydrolytisch spaltet. Das auf diese Weise erhältliche Sulfonamid-Kupplungsfragment weist somit eine endständige $-SO_2-NH_2$ Gruppe auf, an die jetzt sehr einfach ein geeignetes Brückenglied angekoppelt werden kann, das für die nachfolgende Konjugation des Haptenmoleküls einsetzbar ist. Die Ankopplung des Brückenglieds kann beispielsweise dadurch erreicht werden, dass man die zuvor erwähnte $-SO_2-NH_2$ Gruppe mit einem reaktionsfähigen Carbonsäurederivat wie z.B. einem Bersteinsäurederivat, oder aber vorzugsweise mit dem entsprechenden Anhydrid umsetzt. Bei Verwendung eines Bernsteinsäurederivates entsteht dabei die Gruppe $-SO_2-NH-C(O)-CH_2CH_2COOH$. Die Umsetzung wird vorzugsweise in basischem Milieu durchgeführt, ganz besonders bevorzugt in Gegenwart eines geeigneten Katalysators wie beispielsweise des in Beispiel 1.1 verwendeten 1,8-Diazabicyclo[5.4.0.]undec-7-en.

Das erfindungsgemässe Molekülfragment kann selbstverständlich aus ausgehend von bekannten Ausgangsverbindungen und Reaktanten durch chemische Synthese hergestellt werden.

Die endständige Carboxylgruppierung kann anhand bekannter, in der Literatur vielfach beschriebener Verfahren auch in eine Amino- oder eine SH-Gruppe überführt werden, die ebenfalls über eine für die Ankopplung des Haptenmoleküls ausreichenden Reaktivität verfügen.

Als Brückenglieder für eine Konjugation des Haptens an das Carriermolekül kommen somit in erster Linie Verbindungen in Betracht, die mindestens eine oder aber mehrere reaktive Gruppen enthalten, welche in der Lage sind mit den frei zugänglichen reaktiven Gruppen des Carrier-Moleküls in Wechselwirkung zu treten.

Besonders bevorgzugt im Rahmen dieser Erfindung ist die Verwendung von Brückengliedern die zwischen 2 und 10 Brücken-C-atome umfassen und die als reaktive Gruppe(n) eine oder mehrere reaktive Gruppen, wie z.B. Amino-, Carboxyl- oder SH-Gruppe(n) besitzen. Diese reaktiven Gruppen können mit Hilfe an sich bekannter Verfahren mit den reaktiven Gruppen des Hapten- und Carrier-Moleküls zur Reaktion gebracht werden, unter Ausbildung eines Hapten-Carrier-Konjugates.

So ist es beispielsweise möglich ein Brückenglied über eine reaktive Aminogruppe mit Hilfe von Dialdehyden (z.B. Glutardialdehyd) an eine der freien Aminogruppen des Carrier-Moleküls zu binden. Besitzt das Brückenglied eine reaktive SH-Gruppe, so kann die Konjugation des Haptens an das Carrier-Molekül durch eine Oxidation mit freien SH-Gruppen des Carriers durchgeführt werden.

Besonders bevorzugt im Rahmen dieser Erfindung ist die Verwendung von Brückengliedern mit einer Carboxylgruppe, die mit Hilfe von wasserbindenden Mitteln, wie beispielsweise einem Carbodiimid, vorzugsweise N,N'-Dicyclohexylarbodiimid, mit einer freien Aminogruppe des Carriermoleküls verknüpft werden kann.

Um das Antigen an das Trägerprotein zu kuppeln, ist es somit vorteilhaft zunächst ein zu dieser Kupplung befähigtes Derivat herzustellen.

In einer bevorzugten Ausführungsform der vorliegenden Erfindung verwendet man zur Herstellung von Konjugaten, die nach Immunisierung eines Donortieres zu einer spezifischen Immunantwort und letztlich zur Herstellung von monoklonalen Antikörpern mit hoher Affinität gegenüber Sulfonylharnstoff-Herbiziden der Formel (A) führen, ein Fragment, das insbesondere den Sulfonamidteil von besagtem Sulfonylharnstoff-Herbizid umfasst. Nach Derivatisierung dieses Fragments erhält man eine geradezu ideale Kupplungskomponente, die sich in hervorragender Weise für die Herstellung monoklonaler Antikörper mit einer hohen Affinität gegenüber Sulfonylharnstoff-Herbiziden der Formel (A) eignen.

Besonders bevorzugt sind Sulfonylharnstoff-Derivate der Formel (III),

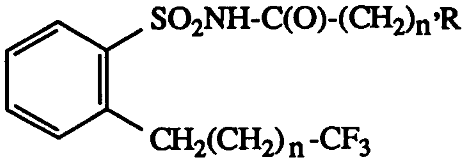

(III)

die man sich durch Austausch der Triazinamin-Gruppierung gegen eine $R'-(CH_2)_{n'}$-Gruppe entstanden denken kann und worin

R für COOH, $NH_2$ oder SH, insbesondere aber für COOH steht; und
n' eine ganze Zahl von 1 bis 10, vorzugsweise von 1 bis 6; und
n eine ganze Zahl von 0 bis 4, vorzugsweise aber von 1 repräsentiert, und die somit auf den Sulfonamidanteil beschränkt sind.

Bevorzugt im Rahmen dieser Erfindung ist eine Kupplungskomponente, worin R für $NH_2$ steht und n' eine ganze Zahl von 2 bis 6, insbesondere aber 3 repräsentiert.

Besonders bevorzugt ist eine Kupplungskomponente, worin R für COOH steht und n' eine ganze Zahl von 1 bis 6, vorzugsweise aber 2 repräsentiert.

Ganz Besonders bevorzugt im Rahmen dieser Erfindung ist das Derivat der Formel (IV),

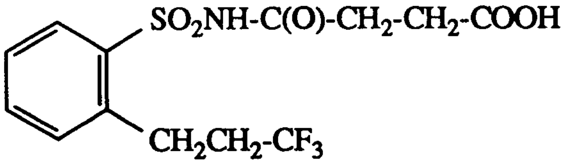

(IV)

das man sich durch Austausch der Triazinamin-Gruppierung gegen eine $-(CH_2)(CH_2)COOH$ Gruppierung entstanden denken kann, und das gemäss dem in Beispiel 1.1 beschriebenen Verfahren hergestellt werden kann. Die in diesem Verfahren verwendbaren Ausgangsverbindungen und Reaktanten sind bekannt oder können analog zu bekannten strukturähnlichen Verbidnungen hergestellt werden.

Die resultierenden, zur Kupplung befähigten Sulfonylharnstoff-Derivate sind neu und stellen durch ihre spezifische Kupplungsfähigkeit ein wertvolles Ausgangsmaterial bei der Herstellung der erfindungsgemässen monoklonalen Antikörper dar. Sie sind daher ein wichtiger Bestandteil vorliegender Erfindung.

Die Durchführung der eigentlichen Kupplungsreaktion erfolgt je nach verwendetem Sulfonylharnstoff-Derivat vorzugsweise mit Hilfe der aktiven Estermethode oder dem Diazoniumverfahren [Kelly *et al* (1985)]. Bei Verwendung der aktiven Estermethode wird das Sulfonylharnstoff-Derivat zunächst in einem geeigneten Lösungsmittel solubilisiert. Als geeignete Lösungsmittel kommen insbesondere aprotische Lösungsmittel, die eine geringe Verdampfungsrate aufweisen, wie z.B. N,N-Dimethylformamid (DMF) oder Dimethylsulfoxid (DMSO) in Betracht.

Anschliessend werden die Carboxylgruppen zu einem aktiven Ester derivatisiert, indem das zuvor solubilisierte Sulfonylharnstoff-Derivat z.B. mit N-Hydroxysuccinimid, N-Hydroxysulfosucclnimid, N,N'-Dicyclohexylcarbodiimid oder N,N'-Carbonyldiimidazol oder mit Derivaten dieser Verbindungen zur Reaktion gebracht wird.

Der aktive Ester wird dann aus dem Reaktionsgemisch abgetrennt und zu BSA oder KLH zugegeben. Nach einer Inkubationszeit von 0.1 bis 12 Stunden vorzugsweise von 4 bis 5 Stunden wird das Präzipitat entfernt. Der Ueberstand kann dann gegebenenfalls nach Zwischenschaltung weiterer Reinigungsschritte für die eigentliche Immunisierungsreaktion verwendet werden.

Neben der im Rahmen dieser Erfindung bevorzugten aktiven Estermethode, können auch alternative Verfahren für die Kupplung des Haptens an das Carrier-Molekül verwendet werden, wie z.B. die gemischte Anhydridmethode. Dabei wird die Carboxylgruppe des Brückengliedes unter Verwendung von Essigsäureanhydrid oder des Carbodiimidderivates 1-Ethyl-3-(3'-dimethylaminopropyl)carbodiimid an das Carrier-Molekül geknüpft.

Erfolgt die Kopplung des Derivates über eine reaktive $NH_2$-Gruppe, so kommt vorzugsweise das bei Kelly *et al* (1985) beschriebene Diazonium-Verfahren zur Anwendung.

Dabei wird die entsprechende reaktive $NH_2$-Gruppe vorzugsweise bei niedriger Temperatur, vorzugsweise bei einer Temperatru um 0°C, in stark saurer Lösung mit Natriumnitrit versetzt. Das resultierende Diazoniumsalz wird dann langsam zu einer gepufferten, basichen Lösung des Carriermoleküls gegeben. Diese Lösung kann dann, gegebenenfalls nach Zwischenschaltung weiterer Reinigungsschritte, für die eigentliche Immunisierung verwendet werden.

Die Immunisierung der Donortiere erfolgt durch eine ein- bis mehrmalige Applikation des an ein hochmolekulares Trägermolekül gekoppelten Haptens. Besonders bevorzugt ist eine 2 bis 3 malige Applikation, die in Abständen von 7 bis 30 Tagen insbesondere aber von 12 bis 16 Tagen erfolgt.

Die im Rahmen der vorliegenden Erfindung bevorzugte Applikationsform ist die Injektion, die sowohl intravenös, intraperitoneal als auch subcutan erfolgen kann. Bevorzugt ist eine Kombination von subcutaner und intraperitonealer Injektion.

Das Immunogen (Sulfonylharnstoff-konjugat) liegt in diesem Fall in einem geeigneten Puffer vor, wie z.B. einem PBS-Puffer, der eines der üblicherweise verwendeten Adjuvantien enthält. Besonders bevorzugt im Rahmen dieser Erfindung ist die Verwendung von Freund's Adjuvans.

Nach einer Ruheperiode von 0,5 bis 4 Monaten erfolgt eine weitere einmalige Applikation ["boost"] des Haptenkonjugates in einer Dosierung von 10 µg bis 1000 µg, insbesondere aber von 50 µg bis 500 µg.

In einem Zeitraum von 1 bis 6 Tagen nach der letzten Dosierung werden die Donortiere getötet, die Milz wird entnommen und es wird eine Milzzellensuspension hergestellt.

Die isolierten Milzzellen werden dabei in einem geeigneten Puffer (z.B. einem BSS-Puffer) suspendiert und bis zu ihrer Fusion mit geeigneten Myeloma-Zellen in Form einer Zellsuspension aufbewahrt.

Anfänglich wurden diese Fusionen dadurch kompliziert, dass auch die Myeloma-Zellinien monoklonale Antikörper synthetisierten, so dass das Hybrid zwei Typen monoklonaler Antikörper produzierte, einen, der seinen Ursprung in der Myeloma-Zelle hatte und einen zweiten, der durch die genetische Information der immunkompetenten Zelle bestimmt wurde.

Im Rahmen der vorliegenden Erfindung werden daher vorzugsweise solche Tumorzellen verwendet, die selbst keine monoklonale Antikörper herstellen können, wie z.B. SP2/0-Ag14 (Shulman *et al*, 1978), X63-Ag8.653 oder PAI [Stocker *et al* (1982)], wodurch die Analyse der resultierenden Fusionsprodukte sehr stark vereinfacht wird. Für den Fusionserfolg ist es vorteilhaft, wenn die Milzzellen in einem 2 bis 20fachen Ueberschuss im Verhältnis zu den Myelomzellen vorliegen.

Die Fusion von Milz- und Myelomzellen wird in einem speziellen Fusionsmedium durchgeführt, das eine Zusammensetzung aufweist, die für die beabsichtigte Zellfusion optimale Voraussetzungen liefert.

Vorzugsweise handelt es sich bei besagtem Fusionsmedium um eine Pufferlösung, die einen der üblicherweise für die Fusionierung von Zellen verwendeten Fusionspromotoren enthält, wie z.B. Sendai Viren oder andere Paramyxoviren, gegebenenfalls in UV-inaktivierter Form, Calcium-Ionen, oberflächenaktive Lipide, wie z.B. Lysolecithin oder Polyethylenglykol. Besonders bevorzugt im Rahmen dieser Erfindung ist die Verwendung von Polyethylenglykol (PEG), insbesondere von Polyethylenglykol (PEG) mit einem mittleren MG von 600 bis 6000, und in einer Konzentration von 30 % bis 60 %. Besonders bevorzugt ist PEG 4000 mit einer Konzentration von ca.50 %. Die optimale Fusionstemperatur beträgt zwischen 18°C und 39°C. Besonders bevorzugt ist eine Temperatur von 37°C.

Nach der erfolgten Fusion der immunkompetenten Milzzellen mit den Myeloma-Zellen werden die fusionierten Antikörper-produzierenden Hybridzellen mit Hilfe an sich bekannter Verfahren selektioniert.

Für das Auslesen erfolgreicher Fusionsereignisse (Hybridzellen) aus den 2 Typen von Elternzellen existieren verschiedene Möglichkeiten. Routinemässig werden eine Million und mehr Zellen von jedem Elterntyp in dem Fusionsprotokoll verwendet. Da die Fusion nicht mit einer 100 %igen Frequenz erfolgt, kann es zu einem schwierigen Unterfangen werden, die Fusionsprodukte von der grossen Ueberzahl nicht fusionierter oder mit sich selbst fusionierter Elternzellen abzutrennen.

Wie bereits zuvor erwähnt, entstehen die Hybridomazellen durch Fusion kurzlebiger Antikörper produzierender (Milz) B-Zellen sowie langlebiger Myeloma Zellen.

Das gewünschte Resultat ist eine langlebige Zellinie, die Antikörper produziert. Da die Milzzellen nur eine begrenzte Lebenszeit in Kultur besitzen, kann man daher im Prinzip einfach abwarten, bis alle nicht-fusionierten sowie alle mit sich selbst fusionierten Milzzellen abgestorben sind. Dann bleibt jedoch immer noch die Aufgabe bestehen, die

langlebigen Antikörper-produzierenden Zellen von den langlebigen nicht Antikörper-produzierenden Zellen abzutrennen.

Ein gängiges Selektionssystem basiert auf der Verfügbarkeit oder Nichtverfügbarkeit des Enzyms Hypoxanthinguaninphosphoribosyltransferase (HGPRT). Dieses Enzym ist Bestandteil des Purin "Salvage Pathway" in Säugerzellen. Diese Zellen sind darüberhinaus auch in der Lage Purine *de novo* zu synthetisieren.

Unter normalen Umständen arbeiten wahrscheinlich beide Synthese-Wege bis zu einem gewissen Grade parallel.

Falls eine Zelle jedoch kein HGPRT besitzt, ist der "Salvage Pathway" blockiert und die Purine müssen aus nicht-Purin Material hergestellt werden.

Für die Selektion HGPRT-negativer Myelomazellen verwendet man in der Regel sog. Purin-Antimetaboliten, wie z.B. das 8-Azaguanin, das dem Purin Guanin strukturell sehr ähnlich ist und dieses daher in einigen seiner normalen Reaktionen ersetzen kann.

Azaguanin wird in die DNA eingebaut, was zu einer Beeinträchtigung des normalen Wachstumsverhaltens und schliesslich zum Zelltod führt. Da Azaguanin über den "Salvage Pathway" ersetzt werden muss, können alle diejenigen Zellen, die keine HGPRT Aktivität besitzen, Azaguanin nicht verwerten und daher in dessen Gegenwart wachsen.

Ein selektives System, das mit demselben Enzym aber unter umgekehrten Vorzeichen arbeitet, indem in diesem Fall HGPRT positive Zellen selektiert werden, ist bei J.W. Littlefield (1964) beschrieben.

Dieses Selektionssystem basiert auf der Verwendung des sogenannten HAT-Mediums, das u.a. Hypoxanthin, Aminopterin und Thymidin (HAT-Medium) als Bestandteile enthält. Aminopterin ist ein Antimetabolit, der die *de novo* Purin-Synthese sowie die Methylierung von Desoxyuridylat zu Thymidylat hemmt.

Hypoxanthin kann als Hilfspurin fungieren für den Fall, dass Aminopterin die *de novo* Purin-Synthese blockiert, während Thymidin die Notwendigkeit einer Methylierung von Desoxyuridylat überflüssig macht.
Daher werden in Gegenwart von Aminopterin alle HGPRT positiven Zellen proliferieren, während die HGPRT negativen Zellen absterben.

In dem Hybridsystem, das im Rahmen dieser Erfindung für die Selektion verwendet wird, sind die Myeloma-Zellen vorzugsweise resistent gegenüber Azaguanin und empfindlich gegenüber Aminopterin, d.h. sie sind HGPRT negativ. Die Antikörper-produzierenden Zellen dagegen sind HGPRT positiv.

Durch Fusion der Zellen und Kultivierung in einem HAT-Medium, können die erfolgreich miteinander fusionierten Zellen selektiert werden, da die Myeloma-Zellen, die für die Proliferation verantwortlich sind, nur in Gegenwart einer HGPRT-Aktivität wachsen können und diese Aktivität von der HGPRT-positiven Zellinie geliefert werden muss.

Die HGPRT-positiven Antikörper-produzierenden Zellinien werden in diesem Medium zwar nicht abgetötet. Sie überleben aber nur eine bestimmte Zeit, und sind nicht in der Lage zu proliferieren.

Damit wird durch Fusion der Zellen in einem HAT-Medium ein System geschaffen, in welchem zwar die Myeloma Zellen und die Antikörper produzierenden Zellen für einen Zeitraum wachsen können, der ausreicht für die Produktion von Hybrid-Zellen, in dem aber nur die Hybrid-Zellen überleben und proliferieren können.

In einer besonderen Ausführungsform der vorliegenden Erfindung werden die fusionierten Hybridzellen in Gegenwart von zuvor aus dem Peritoneum unbehandelter nicht-immunisierter Versuchstiere isolierten Macrophagen, sogenannter "Feederzellen", kultiviert. Für die Kultivierung und die Selektion der fusionierten Hybridzellen wird die Zellsuspension in mehrere Aliquots aufgeteilt und die einzelnen Aliquots werden ständig auf die Ausbildung von Hybridzellkulturen sowie auf die Bildung von Antikörpern hin untersucht.

Besonders bevorzugt im Rahmen dieser Erfindung ist die Kultivierung der fusionierten Hybridzellen auf Mikrotiterplatten.

Dabei wird die nach der Fusion erhaltene Zellsuspension auf die einzelnen Vertiefungen einer Mikrotiterplatte verteilt und für einen Zeitraum von 7 bis 30 Tagen unter geeigneten, das Wachstum der fusionierten Hybridzellen fördernden Bedingungen (z.B. HAT/HT-Medien) kultiviert.
Die Ueberstände gewachsener Hybridkulturen werden ständig auf die Bildung von Antikörpern hin untersucht.

Positive Hybridzellkulturen werden dann mit Hilfe bekannter Verfahren, vorzugsweise aber mit Hilfe des limitierenden Verdünnungsverfahrens vereinzelt und anschliessend als Reinkulturen in geeigneten Kultivierungsmedien kloniert.

Die Ueberstände der gewachsenen Zellklone werden ebenfalls auf die Bildung von Antikörpern hin überprüft.

Das Screening der gemäss der vorangegangenen Beschreibung hergestellten erfindungsgemässen Hybridoma-Zellklone auf die Bildung geeigneter monoklonaler Antikörper erfolgt vorzugsweise mit Hilfe eines der üblicherweise für diesen Zweck verwendeten Immunassays, wie z.B. einem Enzym-gekoppelten Immunassay oder einer Radioimmunassay.

Beim Enzym-gekoppelten Immunassay werden die zuvor näher charakterisierten Hapten-Konjugate zunächst an einen festen Träger adsorbiert. Die verbleibenden freien Bindungsstellen werden anschliessend durch Zugabe von Carriermolekülen abgesättigt und damit blockiert.

Zum Nachweis monoklonaler Antikörper werden Aliquots der Ueberstände besagter Hybridoma-Zellklone mit den Träger-gebundenen Haptenkonjugaten inkubiert.

Ein weiterer Gegenstand der vorliegenden Erfindung betrifft die Herstellung monoklonaler Antikörper unter Verwendung an sich bekannter Verfahren, die dadurch gekennzeichnet sind, dass man die zuvor näher charakterisierten

erfindungsgemässen Hybridomazellinien oder aber Klone oder Subklone davon, welche die erfindungsgemässen Antikörper synthetisieren und vorzugsweise in das umgebende Medium sezernieren, *in vitro* oder *in vivo* mit Hilfe bekannter Verfahren kultiviert.

Die *in-vitro*-Kultivierung der erfindungsgemässen Hybridoma-Zellen erfolgt in geeigneten Kultivierungsmedien, insbesondere in den üblicherweise verwendeten, standardisierten Kulturmedien wie z.B. Dulbecco's Modified Eagle Medium (DMEM) oder RPMI 1640 Medium, die gegebenenfalls durch Zugabe von Säuger-Seren, wie z.B. Foetales Kälberserum, oder durch wachstumsfördernde Zusätze und Spurenelemente ergänzt werden können.

Die Isolierung der monoklonalen Antikörper beginnt vorzugsweise mit einer Präzipitation der Immunglobulinfraktion aus den jeweiligen Ueberständen der Hybridomakulturen, z.B. mit Hilfe von Ammoniumsulfat. Es schliessen sich weitere Aufarbeitungs- und Reinigungsschritte an, die dem Fachmann auf diesem Gebiet bekannt sind und die beispielsweise die Verwendung chromatographischer Methoden, wie z.B. Gelfiltration, Ionenaustausch-Chromatographie, DEAE-Cellulose Chromatographie, Protein A oder Immunaffinitätschromatographie miteinschliessen.

Grosse Mengen der erfindungsgemässen monoklonalen Antikörper können aber auch mit Hilfe von *in vivo* Verfahren gewonnen werden.

So ist es beispielsweise möglich Antikörper-produzierende Hybridoma-Zellklone in geeignete Säugetiere zu injizieren, welche die Entwicklung von Antikörperproduzierenden Tumoren in den behandelten Tieren induzieren. Nach einem Zeitraum von 1 bis 3 Wochen können die Antikörper aus den Körperflüssigkeiten der so behandelten Tiere isoliert werden.

In einer besonderen Ausführungsform der vorliegenden Erfindung wird weiblichen Balb/c Mäusen, die gegebenenfalls mit einem Kohlenwasserstoff wie z.B. Pristan vorbehandelt wurden, ein erfindungsgemässer Hybridoma-Zellklon intraperitoneal injiziert.

Ein bis drei Wochen nach der Injektion des Hybridoma-Zellklons wird die Ascitesflüssigkeit gesammelt und bis zur weiteren Aufbereitung aufbewahrt.

Die Isolierung der monoklonalen Antikörper erfolgt in genau analoger Weise zu der zuvor beschriebenen Isolierung aus den Ueberständen *in vitro* kultivierter Hybridomas.

Ein weiterer Gegenstand der vorliegenden Erfindung betrifft die Verwendung der erfindungsgemässen Antikörper in einem der gebräuchlichen Immunassays zum Nachweis von Sulfonylharnstoff-Herbiziden der Formel (A), vorzugsweise aber zum Nachweis von Sulfonylharnstoff-Herbiziden der Formel (I) und ganz besonders bevorzugt zum Nachweis einer Verbindung gemäss Formel (II), sowie zur Differenzierung von besagten Sulfonylharnstoff-Herbizidengegenüber strukturell verwandten Verbindungen, wie z.B. Triasulfuron, Primisulfuron und Chinosulfuron sowie der Verbindung A in Tabelle 2.

Die erfindungsgemässen monoklonalen Antikörper können somit in allen bekannten Immunassays verwendet werden, die auf der spezifischen Bindung zwischen Antigen und dem entsprechenden monoklonalen Antikörper aufbauen, wie z.B. in einem Radioimmunassay (RIA), einem Enzym-gekoppelten Immunassay (ELISA), einem Immunfluoreszenz-Test etc.

Beim RIA Test kann der erfindungsgemässe monoklonale Antikörper als solcher oder aber in Form eines radioaktiv markierten Derivates verwendet werden. Dabei können alle bis heute bekannten Modifikationen des RIA Tests für den Nachweis der im Rahmen dieser Erfindung relevanten Zielsubstanzen verwendet werden, wie z.B. ein RIA Test in homogener oder fester Phase, ein heterogener RIA Test sowie ein einfacher RIA Test mit direktem oder indirektem (kompetitiven) Nachweis des Antigens. Das gleiche gilt auch für die Verwendung eines enzymgekoppelten Immunassays.

Bevorzugt im Rahmen dieser Erfindung ist die Verwendung eines erfindungsgemässen monoklonalen Antikörpers in einem kompetitiven Immunassay zum Nachweis von Sulfonylharnstoff-Herbiziden der Formel (I), insbesondere aber zum Nachweis einer Verbindung gemäss Formel (II).

Das Prinzip des kompetitiven Immunassays beruht auf einer Konkurrenz zwischen einem markierten bzw. einem an einen festen Träger gebundenen Antigen und einem freien Antigen um die relevanten Bindungsstellen auf dem Antikörpermolekül.

Grundsätzlich unterscheidet man zwei Möglichkeiten zur Durchführung dieses kompetitiven Immunassays.

a) Das erste Verfahren beruht auf der Konkurrenz zwischen dem an einen festen Träger gebundenen Antigen und freiem Antigen um die freien Bindungsstellen auf dem Antikörper, der mit einem Marker versehen ist. Die Bindung des Antigens an einen festen Träger kann dabei entweder direkt oder aber über ein Carriermolekül erfolgen.

Die Bestimmung der Konzentration an Ziem Antigen erfolgt in diesem Fall über die Abnahme des markierten, an das Träger-fixierte Antigen gebundenen Antikörpers. Diese Abnahme ist proportional der in der Probe enthaltenen Menge an freiem Antigen.

b) Ein alternatives Verfahren basiert darauf, dass freies und markiertes Antigen miteinander um die relevanten Bindungsstellen des Antikörpers konkurrieren, der in diesem Fall an einen festen Träger gebunden vorliegt.

Die Bestimmung der Konzentration an freiem Antigen erfolgt über die Abnahme an markiertem Antigen, die in Abhängigkeit von der Konzentration an freiem Antigen variiert.

Als festes Trägermaterial, das für die Bindung des Antigens oder des Antikörpers geeignet ist, kommen beispielsweise die Plastikoberfläche einer Mikrotiterplatte oder eines Teströhrchens, die Oberfläche von Kugeln aus Polystyrol, Polypropylen, Polyvinylchlorid, Glas oder Kunststoff oder aber die Oberfläche von Filterpapier-, Dextran-Cellulose- oder Nitrozellulose-Streifen oder ähnliche Materialien in Betracht. Diese werden mit einem der erfindungsgemässen monoklonalen Antikörper oder einem Antigen überzogen, wobei die Bindung an das Trägermaterial durch einfache Adsorption oder aber gegebenenfalls nach vorangegangener Aktivierung des Trägermaterials mit z.B. Glutaraldehyd oder Cyanogenbromid vermittelt werden kann.

Besonders bevorzugt im Rahmen dieser Erfindung ist die Verwendung des erfindungsgemässen monoklonalen Antikörpers in einem Enzym-gekoppelten Immunassay [ELISA ('Enzyme Linked Immuno Sorbent Assay)]. Dabei kann der erfindungsgemässe monoklonale Antikörper als solcher oder in Form eines Enzym-gekoppelten Derivates verwendet werden.

Der ELISA Assay basiert entweder auf der Verwendung eines Enzym-gekoppelten Derivates des erfindungsgemässen Antikörpers oder aber von an sich bekannten Enzym-gekoppelten Antikörpern, die ein Epitop eines erfindungsgemässen Antikörpers erkennen und daran binden.

Besonders bevorzugt im Rahmen dieser Erfindung ist die Verwendung eines ELISA-Assays, bei dem eines der zuvor beschriebenen Trägermaterialien zunächst mit einem Antigen beschichtet wird. Das trägergebundene Antigen wird anschliessend mit einer Testlösung inkubiert, die das nachzuweisende Antigen sowie einen der erfindugsgemässen Antikörper enthält. Das nachzuweisende Antigen kann dabei entweder in freier Form oder aber als Bestandteil einer Wasser- oder Bodenprobe vorliegen.

Nach einer Inkubationszeit von 10 Minuten bis 2 Stunden wird der gesamte Ansatz mit einem enzymmarkierten Antikörper inkubiert, der den erfindungsgemässen monoklonalen Antikörper erkennt und an diesen bindet. Ein Beispiel eines solchen enzymmarkierten Antikörpers ist ein Phosphatase-markiertes Ziegen anti-Schaf Immunglobulin, oder ein entsprechender Ziegen anti-Maus Antikörper, die kommerziell bezogen werden können.

Die Menge des gebundenen Antikörperproteins lässt sich anhand einer Enzym-Substrat-Reaktion, z.B. mit Hilfe spektroskopischer Verfahren bestimmen.

Ebenfalls bevorzugt im Rahmen dieser Erfindung ist ein ELISA-Test, der auf der Konkurrenz von markiertem sowie von freiem Antigen um den an eines der zuvor genannten Trägermaterialien gebundenen Antikörper beruht.
Der Anteil an freiem Antigen, der in einer bestimmten Probe vorliegt, wird in diesem Fall über die Abnahme an markiertem Antigen bestimmt, die umso präziser ist, je mehr freies Antigen die Probe enthält.

Die vorliegende Erfindung betrifft weiterhin Mittel für die qualitative und quantitative Bestimmung von Sulfonylharnstoff-Herbiziden der Formel (A), vorzugsweise aber zur Bestimmung von Sulfonylharnstoff-Herbiziden der Formel (I) und ganz besonders bevorzugt zur Bestimmung einer Verbindung der Formel (II), in Form eines Test-Kits, die neben den erfindungsgemässen monoklonalen Antikörpern und/oder deren Derivaten, gegebenfalls auch noch andere monoklonale oder polyklonale Antikörper, insbesondere aber markierte monoklonale oder polyklonale Antikörper, sowie weitere Zusätze enthalten können.

Besonders bevorzugt im Rahmen dieser Erfindung sind Test-Kits, die auf einem der üblicherweise verwendeten Immunassays basieren, ausgewählt aus der Gruppe bestehend aus Radioimmunassay, Enzym-gekoppelter Immunassay und Chemilumineszenzassay. Ganz besonders bevorzugt sind Test-Kits, bei denen der Nachweis der Zielsubstanz auf einem kompetitiven Immunassay, insbesondere aber auf einem Enzym-gekoppelten Immunassay (ELISA) beruht.

Test-Kits für einen radioimmunologischen Nachweis einer Verbindung gemäss Formel (II), die im Rahmen dieser Erfindung bevorzugt sind, können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines radioaktiv markierten Derivates davon oder radioaktiv markiertes Antigen oder standardisierte Lösungen des Antigens;
(c) Pufferlösungen und
(d) gegebenenfalls Polypeptide, Detergentien und weitere Zusätze, die beispielsweise eine unspezifische Adsorption und Aggregatbildung verhindern sowie
(e) Pipetten, Reaktionsgefässe, Eichkurven, Beipackzettel etc.

Test-Kits für den immunologischen Nachweis einer Verbindung gemäss Formel (II), die auf einem Enzym-gekoppelten Immunassy (ELISA) basieren, können beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und/oder eines zweiten Enzym-markierten monoklonalen oder polyklonalen Antikörpers, der gegen das zu bestimmende Antigen oder einen das Antigen erkennenden Antikörper gerichtet ist;
(c) Enzymsubstrate in fester oder gelöster Form;
(e) das Antigen oder standardisierte Lösungen des Antigens;
(f) Pufferlösungen;
(g) gegebenenfalls Polypeptide, Detergentien und weitere Zusätze, die beispielsweise eine unspezifiche Adsorption und Aggregatbildung verhindern sowie
(h) Pipetten, Reaktionsgefässe, Eichkurven, Farbtafeln, Beipackzettel, etc.

Ein Test-Kit für den Nachweis von einer Verbindung gemäss Formel (II), der auf einem Chemiluineszenztest basiert, kann beispielsweise die folgenden Bestandteile enthalten:

(a) ein geeignetes Trägermaterial, das unbeschichtet oder aber mit einem der erfindungsgemässen Antikörper oder einem Antigenkonjugat beschichtet sein kann;
(b) gegebenenfalls gefriergetrocknete oder konzentrierte Lösungen eines der erfindungsgemässen Antikörper und eines zweiten polyklonalen Antikörpers, der in der Lage ist, den erfindungsgemässen ersten Antikörper zu erkennen und der mit einem chemilumineszierenden Marker verknüpft ist;
(c) Lösungen enthaltend eine Komponente, die die Emission von Licht auslöst, wie z.B. $H_2O_2$ und NaOH;
(d) Pufferlösungen;
(e) gegebenenfalls Polypeptide, Detergentien und weitere Zusätze, die eine unspezifische Adsorption und Aggregatbildung verhindern sowie
(f) Pipetten, Reaktionsgefasse, Beipackzettel, etc.

Trägermaterialien, die im Rahmen der vorliegenden Erfindung Verwendung finden können, umfassen in erster Linie unlösliche, polymere Materialien, ausgewählt aus der Gruppe bestehend aus Polystyrol, Polyethylen, Polypropylen, Polyester, Polyacrylnitril, Polyvinylchlorid, Polyacrylamid, Nitrocellulose, quervernetztes Dextran, fluorierte Harze, Agarose, quervernetzte Agarose, Polysaccharide, etc. Daneben sind aber auch andere Materialien denkbar, wie z.B. Glas, Metall, Netzgewebe auf Nylonbasis, etc.

Die zuvor im einzelnen genannten Trägermaterialien können sehr unterschiedlich ausgestaltet sein und in Abhängigkeit vom jeweils angestrebten spezifischen Verwendungszweck sehr verschiedenartige Formen aufweisen. Diese umfassen beispielsweise Schalen, Kugeln, Platten, Stäbchen, Zellen, Fläschchen, Röhrchen, Fasern, Netze, etc.

Häufige Verwendung bei der Herstellung von Test-Kits finden beispielsweise Mikrotiterplatten aus durchsichtigen Plastikmaterialien, wie z.B. Polyvinylchorid oder Polystyrol, die unbeschichtet oder aber mit einem der erfindungsgemässen Antikörper, mit freiem Antigen oder einem Antigenkonjugat beschichtet sein können. Ebenso verwendet werden Kügelchen, Röhrchen oder Stäbchen aus Polystyrol sowie Polystyrollatex, wobei das umgebende Latexmaterial via Zentrifugation von den Polystyrolpartikeln abgetrennt werden kann.

Einen weiteren Bestandteil des erfindungsgemässen Teskits bilden Marker oder Indikatoren, mit deren Hilfe es möglich ist, das Vorliegen einer Komplexbildungsreaktion, insbesondere aber einer Immunreaktion nachzuweisen, die vorzugsweise zu einem Antigen-Antikörper-Komplex oder aber zu einem Ligand-Rezeptor-Komplex führt, wobei neben qualitativen gegebenenfalls auch quantitative Aussagen über das nachzuweisende Antigen möglich sind. Als Marker oder Indikatoren kommen sowohl einzelne Atome als auch Moleküle in Betracht, die entweder direkt oder aber indirekt an der Erzeugung eines nachweisbaren Signals beteiligt sein können. Diese Marker oder Indikatoren können entweder direkt mit dem nachzuweisenden Antigen oder mit einem der erfindungsgemässen monoklonalen Antikörper verknüpft sein oder aber in diese eingebaut vorliegen. Sie können aber auch als Einzelsubstanzen oder als Bestandteil einer separaten Verbindung vorliegen, die weder das nachzuweisende Antigen selbst noch einer der erfindungsgemässen monoklonalen Antikörper ist, die ihrerseits aber in der Lage ist mit dem Rezeptormolekül zu reagieren, z.B. in Form einer Komplexbildung.

Bei diesen separat vorliegenden Verbindungen handelt es sich vorzugsweise um ein zweites Antikörpermolekül, das sowohl monoklonalen als auch polyklonalen Ursprungs sein kann, um ein Komplementprotein oder Fragmente davon, um S.*aureus* protein A, etc. Diese separaten Verbindungen erkennen und binden spezifisch an ein Rezeptormolekül, wie z.B. das nachzuweisende Antigen oder einen der erfindungsgemässen monoklonalen Antikörper, vorzugsweise aber an ein Rezeptormolekül, das in Form eines Komplexes vorliegt.

In vielen Fällen sind weitere, zusätliche Reagentien notwendig, die dann erst im Zusammenwirken mit dem Marker zu einem nachweisbaren Signal führen. Dies gilt insbesondere dann, wenn Enzyme involviert sind.

Marker oder Indikatoren, die im Rahmen der vorliegenden Erfindung verwendet werden können, sind dem Fachmann auf dem Gebiet der Immunologie und Immunchemie bestens bekannt. Sie umfassen beispielsweise radioaktiv markierte Elemente oder Substanzen, Enzyme oder chemilumineszierende Substanzen. Die folgende Aufzählung möglicher Marker oder Indikatoren soll lediglich dazu dienen, die grosse Vielfalt der verwendbaren Substanzen und Reagentien beispielhaft zu veranschaulichen, ohne dadurch jedoch den Erfindungsgegenstand in irgendeiner Weise einzuschränken.

Geeignete Marker oder Indikatoren sind beispielsweise innerhalb der Gruppe der radioaktiven Elemente zu finden. Dabei sind insbesondere solche Elemente bevorzugt, die entweder selbst γ Strahlen emittieren, wie z.B. $^{124}$J, $^{125}$J, $^{128}$J, $^{132}$J, $^{51}$Cr oder aber eine Emittierung dieser Strahlen induzieren, wie z.B. $^{11}$C, $^{18}$F, $^{13}$N. Ebenfalls geeignet sind sog. β-Strahler wie $^{111}$In, $^{14}$C und $^{3}$H.

Weitere geeignete Marker umfassen chemilumineszierende Substanzen, insbesondere aber fluoreszierende Substanzen, die sehr einfach auf chemischem Wege mit dem Antigen oder einem Antikörper verbunden werden können ohne diesen zu denaturieren. Das entstehende Fluorochrom lässt sich sehr einfach mit Hilfe fluorometrischer Verfahren nachweisen. Im einzelnen zu nennen sind an dieser Stelle Fluorochrome ausgewählt aus der Gruppe bestehend aus Fluoresceinisocyanat, Fluoresceinisothiocyanat, 5-Dimethylamino-1-naphthalinsulfonylchlorid, Tetramethylrhodanisothiocyanat, Lissamin, Rhodamin 8200 Sulfonylchlorid, etc.

Weitere fluoreszierende Agentien sowie eine Beschreibung von Analysetechniken findet sich bei DeLuca, "Immunofluorescence Analysis", in: Antibody As a Tool, Marchalonis et al, John Wiley & Sons, Ltd., pp 189-231 (1982).

Besonders bevorzugt im Rahmen dieser Erfindung ist die Verwendung von Enzymen als Marker- oder Indikatorsubstanzen, wie z.B. Meerrettichperoxidase, alkalische Phosphatase, β-D-Galaktosidase, Glucoseoxidase, Glucoamylase, Carbonsäureanhydrase, Acetylcholinesterase, Lysozym, Malatdehydrogenase, Glucose-6-phosphatdehydrogenase, etc. Bei der Verwendung von Enzymen als Markersubstanzen ist es notwendig zusätzliche Reagentien zuzugeben, die es erlauben, die Bildung eines Immunkomplexes über die Enzymaktivität zu verfolgen sowie gegebenenfalls ein Stop-Reagenz, mit welchem die Enzymreaktion beendet werden kann.

Besonders bevorzugt sind dabei Reagentien, die zu einer Farbreaktion führen. Im Falle der Meerrettichperoxidase sei an dieser Stelle beispielhaft Hygrogenperoxid genannt, das in Kombination mit einem zusätzliche, oxidierten Farbstoffprecursor wie z.B. Diamino-benzidin oder o-Phenylendiamin, zu einer Braun bzw. Gelbfärbung führt. Bei Verwendung der Glucoseoxidase als Markersubstanz kann beispielsweise 2,2'-Azino-di-(3-ethyl-benzthiazolin-6-sulfonsäure) [ABTS] als Substrat eingesetzt werden.

Ein weiterer Gegenstand der vorliegenden Erfindung betriff somit die Verwendung von Test-Kits, die zumindest einen der erfindungsgemässen monoklonalen Antikörper als Reagenz enthalten, zum schnellen und effektiven, qualitativen und/oder quantitativen Nachweis von Sulfonylharnstoff-Herbiziden der Formel (A), vorzugsweise aber zum Nachweis von Sulfonylharnstoff-Herbiziden der Formel (I) und ganz besonders bevorzugt zum Nachweis einer Verbindung der Formel (II) sowie zur Differenzierung dieser Verbindungen gegenüber strukturell verwandten Verbindungen wie z.B. Triasulfuron, Primisulfuron und Chinosulfuron sowie den Verbindungen A und B in Tabelle 2.

Der erfingungsgemässen monoklonalen Antikörper können darüberhinaus sehr vorteilhaft im Rahmen sog. 'Immunopurification' Analysen eingesetzt werden, die insbesondere bei der Rückstandsanalyse Anwendung finden. Dabei werden die erfindungsgemässen Antikörper kovalent an eine Matrix, vorzugsweise eine Gel-Matrix, gebunden und anschliessend in solcherweise gebunden Form in eine Minisäule plaziert. Diese Säule kann dann für die Reinigung von Bodenextrakten verwendet werden.

Ein weiterer Gegenstand der vorliegenden Erfindung betrifft somit die Verwendung eines der erfindungsgemässen monoklonalen Antikörper in einem 'Immunopurification' Verfahren, die dadurch gekennzeichnet, dass man

(a) besagten Antikörper an einen festen Träger immobilisiert;
(b) besagten Träger in eine Minisäule plaziert;
(c) eine Pufferlösung enthaltend die zu reinigende oder zu analysierende Probe auf die Säule appliziert;
(d) gegebenfalls einen oder mehrere Wasch-Schritte zwischenschaltet; und
(e) den Analyten mit einer geeigneten Elutionslösung von der Säule eluiert.

## I. AUSFÜHRUNGSBEISPIELE

### Beispiel 1 Herstellung des Hapten-Protein Konjugats

Beispiel 1.1: Herstellung von 2-(3,3,3-Trifluorpropyl-phenyl)-N-(1,4-dicarbonsäureamid)-sulfonamid

Die Herstellung eines zur Kupplung an ein Trägerprotein befähigten Derivats einer Verbindung gemäss Formel (II) kann gemäss dem nachfolgenden Reaktionsschema durchgeführt werden:

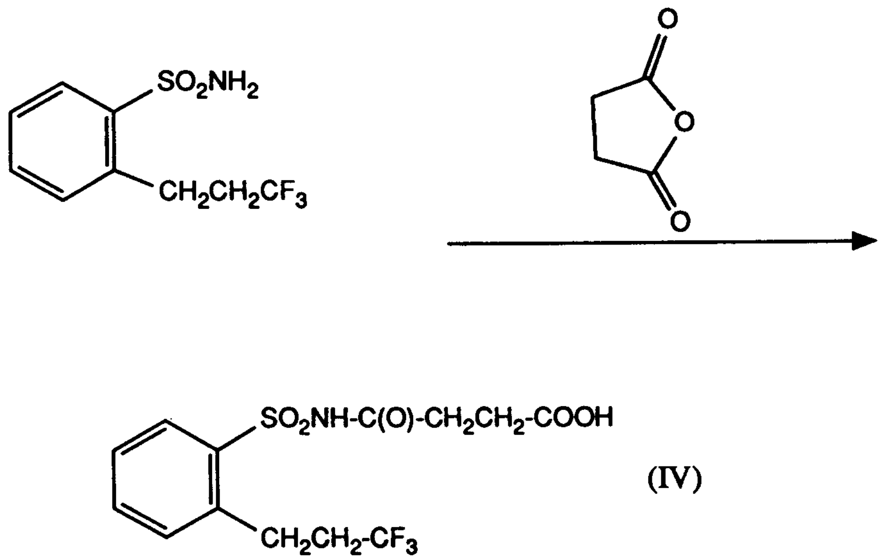

(IV)

Im einzelnen werden die folgenden Verfahrensschritte durchgeführt.

Zu einer Lösung von 25,3 g 2-(3,3,3-Trifluorpropyl)-phenylsulfonamid und 10.0 g Bernsteinsäureanhydrid in 250 ml Dioxan werden unter leichtem Kühlen bei Raumtemperatur über einen Zeitraum von einer halben Stunde 31.4 g 1,8-Diazabicyclo-[5.4.0]undec-7-en, gelöst in Dioxan, zugetropft.

Die resultierende Suspension wird 15 Stunden bei einer Temperatur von 20° bis 25°C weitergerührt, mit 65 ml 2n HCl angesäuert, filtriert und anschliessend getrocknet. Das ausgefallene Produkt wird dann an einer Kieselgel-Säule unter Verwendung von Toluol/Essigester [3:1] als Elutionsmittel gereinigt. Man erhält auf diese Weise 9.5 g der Titelverbindung gemäss Formel (IV), die einen Schmelzpunkt von 138°C bis 139°C aufweist.

Die in dem oben beschriebenen Verfahren verwendeten Ausgangsverbindungen und Reaktanten sind bekannt oder analog bekannter Verfahren herstellbar.

Beispiel 1.2: Hapten-Protein Konjugat

Das gemäss Beispiel 1.1 hergestellte Derivate der Verbindung gemäss Formel (II) wird unter Anwendung der aktivierten Ester-Methode (Kulkarni *et al.*, 1981) entweder an "Bovine Serum Albumine" (BSA; Fluka) oder an "Keyhole Limpet Hemocyanin" (KLH; Calbiochem) konjugiert.

1.2.1: Kupplung der Verbindung der Formel (IV) an BSH oderKLH nach der aktivierten Ester Methode

Im einzelnen wird dabei die Carboxylgruppe der in Formel (IV) wiedergegebenen Verbindung in N,N-Dimethylformamid (DMF) (7.2 mg/200 µl) bei Raumtemperatur solubilisiert und anschliessend mit einem 4 molaren Ueberschuss von N-Hydroxysuccinimid (9.1 mg/200 µl) sowie N,N'-Dicyclohexylcarbodiimid (16 mg/200 µl) versetz. Das Reaktionsgemisch wird zunächst 1 Stunde bei 22°C und anschliessend 18 Stunden bein 4°C gerührt.

Das bei der Reaktion gebildete Präzipitat wird durch 3 minütige Zentrifugation mit 12'000 g bei Raumtemperatur entfernt und der im klaren Überstand vorliegende aktivierte Ester anschliessend zu BSA [12 mg] oder KLH [12 mg] gegeben, das zuvor in 3.3 ml einer Phosphat-gepufferten Salzlösung (PBS-Puffer) solubilisiert worden ist.

Nach 4stündiger Inkubation bei einer Temperatur von 4°C wird das gebildete Präzipitat durch 10 minütige Zentrifugation mit 2'000 g bei 4°C entfernt und der Ueberstand, welcher das Proteinkonjugat enthält, ausgiebig gegen PBS dialysiert, bevor er dann für die Immunisierungs-Experimente verwendet wird.

Das Ausmass der Kupplungsreaktion wird durch Absorptionsspektrophotometrie bestimmt. Das molare Verhältnis von Hapten zu BSA liegt bei ca. 20/1, vorzugsweise aber bei 23/1.

Beispiel 2: Immunisierung

2 Gruppen von jeweils 5 weiblichen, 4 bis 6 Wochen alten BALB/c Mäusen (Tierfarm Sisseln, Schweiz) erhalten in einem Abstand von jeweils 2 Wochen eine Serie von 3 intra-peritonealen bzw. subkutanen Injektionen mit KLH-Konjugat. Die Dosierung für das in Beispiel 1.2.1 hergestellte Konjugat beträgt 25 µg/Injektion.

Die erste Injektion beinhaltet 0,1 ml des Konjugates in PBS, das in einem Verhältnis von 1:1 mit 0,1 ml komplettem

Freund Adjuvanz vermischt ist.

50 µl dieser Injektionslösung werden intraperitoneal injiziert, die restlichen 150 µl subcutan.

Bei der zweiten und dritten Injektionsserie, die 14 bzw. 30 Tage nach der Erstapplikation erfolgt, wird das komplette Freund Adjuvans durch inkomplettes ersetzt.

1 Woche nach der letzten Injektion wird Blutserum von den Versuchstieren entnommen und die Bluttiter mit Hilfe eines ELISA-Tests bestimmt, wobei die Mikrotiterplatten zuvor mit BSA-konjugiertem Hapten beschichtet werden (siehe Abschnitt 6).

Nach einer Ruheperiode von 2 Monaten erfolgt eine weitere einmalige intraperitoneale Injektion der KLH-Konjugate in einer Dosierung von 220 µg/200 µl PBS [Hapten-Protein -Konjugat]. Drei bis vier Tage später werden die Mäuse getötet und die aus diesen isolierten Milzzellen mit der Myelomazellinie PAI [Stocker *et al* (1982)] fusioniert [vergl. Beispiel 3.4].

Beispiel 3: Fusionsprotokoll

3.1. Gewinnung von "Feeder"-Zellen (peritoneale Makrophagen).

Unbehandelte, ca. 6 bis 8 Wochen alte Balb/c-Mäuse werden einen Tag vor der beabsichtigten Fusion getötet und durch Eintauchen in 70 %igem Alkohol sterilisiert.

Anschliessend wird das Fell und die obere Bauchhaut steril angeschnitten ohne das Peritoneum zu verletzen. Mit einer sterilen 5 ml Plastikspritze und einer sterilen 18-er Injektionsnadel werden 4 ml BSS (ohne $Ca^{2+}$ und $Mg^{2+}$) und 1 ml Luft in die Bauchhöhle injiziert.

Nach leichtem Massieren des Bauches (wobei Spritze und Nadel in der Bauchhöhle verbleiben) wird der zuvor injizierte BSS-Puffer wieder aus dem Peritoneum abgezogen und in ein steriles Falconröhrchen gegeben. Dieses Procedere wird noch 2 mal wiederholt. Die so gewonnenen Makrophagen werden mit Eis gekühlt und anschlieessend 2 x mit je 20 ml BSS gewaschen.

Die Makrophagen werden dabei je 10 Min. bei 300 g und einer Temperatur von 5°C zentrifugiert. Das Pellet wird dann in 50 ml HAT-Medium resuspendiert und die Zellsuspension auf 4 Costarplatten mit insgesamt 24 Vertiefungen verteilt (0,5 ml/Vertiefung).

Anschliessend werden die so vorbereiteten Makrophagen bei einer Temperatur von 37°C und einer $CO_2$-Konzentration von 6 % in einem Inkubator aufbewahrt.

Pro Fusionsvorgang werden ca. 4 x $10^6$ Makrophagen benötigt.

3.2. Anzucht der Myeloma-Zellinie PAI

Bei der genannten Myeloma-Zellinie PAI handelt es sich um eine Myeloma-Zellinie, die selbst keine Antikörper sezerniert und die bei Stocker *et al*. (1982) beschrieben ist.

Pro Fusion benötigt man 50 ml einer gut gewachsenen Kultur, die mindestens 10 Millionen Zellen enthält. Die Kultivierung der Myelomzellen erfolgt vorzugsweise in T 175 "Falcon"-Flaschen (Fa. Beckton & Dickenson).

Einen Tag vor der Fusion wird das Kultivierungsmedium (RPMI 1640) durch frisches RPMI 1640-Medium ersetzt. Am Tag der Fusion werden die PAI Zellen geerntet, in ein steriles 50 ml Plastikröhrchen gegeben und 10 Min. bei 300 g und einer Temperatur von 5°C zentrifugiert (MSE Zentrifuge, Modell Chilspin, UK). Nach der Zentrifugation wird der Ueberstand abgezogen und verworfen. Die Zellen werden 2 x mit je ca. 30 ml BSS-Puffer ($Ca^{2+}$ und $Mg^{2+}$ frei) gewaschen (10 Min. bei 300 g, 5°C) und anschliessend in 5 ml BSS resuspendiert.

Ein Aliquot der Zellsuspension wird zur Bestimmung der Zellzahl entnommen und mit Fluoresceindiacetat (FDA) gefärbt. Bis zur Weiterverwendung werden die Myelomzellen auf Eis aufbewahrt.

3.3 Herstellung einer Milzzellsuspension

Die Entnahme der Milz aus einer zuvor gemäss Beispiel 2 immunisierten Balb/c-Maus erfolgt sterilen Bedingungen und unter Kühlung mit Eis.

Die zuvor immunisierte Balb/c-Maus wird durch Genickbruch getötet und die Milz steril entnommen. Dazu wird die Maus kurz in 70 %igen Ethanol eingetaucht und mit sterilem Besteck präpariert. Die Milz wird vorsichtig entnommen und auf ein feines Nylonnetz gelegt. Dort wird sie mit einer Schere fein zerschnitten und dann mit Hilfe eines 5 ml Spritzenstempels vorsichtig durch das Netz gedrückt, ohne dabei zu viele Zellen zu zerstören. Während des ganzen Vorgangs wird das Netz mit BSS gespült.

Die so gewonnene Zellsuspension wird in 50 ml Plastikröhrchen gegeben und 10 Min. bei 300 g und einer Temperatur von 5°C zentrifugiert (MSE Zentrifuge, Modell Chilspin; UK). Die Zellen werden anschliessend 2 x mit je 20 ml BSS gewaschen (10 Min.; 300 g; 5°C; MES Chilspin) und das Zellpellt wird nach der Zentrifugation in 10 ml BSS resuspendiert.

Bis zur Fusion mit PAI Myelomzellen werden die Millzzellen auf Eis belassen.

3.4. Fusion:Millzzellen und PAI Myelomzellen

Das Verhältnis von Myelomzellen zu Milzzellen sollte für die Fusion 1:10 betragen.

Milzzelen (in BSS-Puffer) and PAI Myelomzellen (in BSS-Puffer) werden in dem angegebenen Verhältnis zusammengegeben und 10 Min. bei 300 g und einer Temperatur von 5°C zentrifugiert (MSE Zentrifuge, Modell Chilspin). Das Pellet wird nochmals in BSS-Puffer resuspendiert und die Suspension anschliessend erneut zentrifugiert. Das Pellet wird vorsichtig aufgerührt und in 37°C warmes Wasserbad gestellt. Es wird dann 1 ml vorgewärmtes und steriles PEG-4000 (MERCK) über einen Zeitraum von 60 Sek. tropfenweise zu den Zellen zugegeben, wobei der gesamte Ansatz ständig bewegt wird. Die Zellen werden anschliessend noch 30 Sek. lang weitergeschüttelt, bevor 5 ml einer zuvor erwärmten BSS-Puffers (ohne $Ca^{2+}$, $Mg^{2+}$) ebenfalls tropfenweise über einen Zeitraum von ca. 5 Min. unter ständigem Rühren dazugegeben werden.

Die auf die beschriebene Weise fusionierten Zellen werden dann abzentrifugiert (10 Min.; 300 g; 20°C, MSE Zentrifuge, Modell Chilspin), der Ueberstand wird abgezogen und verworfen. Das Zellpellet wird in 50 ml HAT-Medium resuspendiert und die so erhaltene Zellsuspension auf die vorbereiteten 4 Costarplatten (Mikrotiterplatten mit 24 Vertiefungen, Durchmesser pro Vertiefung 24 mm; Gesamtfläche für Zellwachstum 2,0 $cm^2$) verteilt (0,5 ml/Vertiefung).

Die Inkubation der Costarplatten erfolgt bei einer Temperatur von 37°C und bei einer $CO_2$-Konzentration von 6 %.

Beispiel 4: Kultivierung der Hybridzellen

Am 1. Tag nach der Zellfusion wird 1 ml HAT-Medium pro Vertiefung zu den Kulturplatten zugegeben. 3 bis 4 Tage nach der Zellfusion erfolgt eine mikroskopische Kontrolle der fusionierten Zellen. Gleichzeitig wird das verbrauchte Medium abgesaugt und durch 1 ml frisches HAT-Medium ersetzt. Nach weiteren 3 Tagen (6 - 7 Tage nach der Zellfusion) erfolgt ein erneuter Wechsel des Kulturmediums. Ab dem 7. bis 10. Tag nach der Zellfusion wird jede Vertiefung mikroskopisch nach Hybriden abgesucht und 2 bis 3x wöchentlich das Medium erneuert.

Sobald in einer Vertiefung Hybride gewachsen sind, gewöhnlich nach 2 bis 4 Wochen, kann das HAT Medium dort durch HT-Medium ersetzt werden. Der Ueberstand von gewachsenen Hybridkulturen (mindestens 10 % der Vertiefung) wird mit einer sterilen Pasteurpipette abgehoben und auf das Vorhandensein von Antikörpern getestet.

Sobald die Vertiefung mit positiven Hybridkolonien voll bewachsen sind, können diese auf neue Costarplatten in RPMI 1640-Medium transferriert werden, wobei der Inhalt einer vollbewachsenen Vertiefung auf 2 bis 3 neue Vertiefungen verteilt wird.

Beispiel 5: Klonierung der positiven Hybridzellen

Die Zellen einer positiven Vertiefung werden mit Hilfe einer Pipette gelöst und in 1 ml Medium in ein Röhrchen überführt. Anschliessend wird ein Aliquot zur Bestimmung der Zellzahl entnommen und mit FDA angefärbt (Verdünnung 1 : 2 mit FDA: 50 µl Zellen + 50 µl Farbstoff. Die bevorzugte Zellzahl liegt bei $10^5$ bis $10^6$ Zellen/ml. Anschliessend werden die Hybridzellen in einem Verhältnis von 1 : 100 mit HT-Medium verdünnt (z.B. 100 µl Zellen + 9,9 ml HT-Medium).

In zwei 50 ml Falconröhrchen werden je 25 ml HT-Medium vorgelegt und mit 5 ml einer Macrophagensuspension auf insgesamt 30 ml pro Röhrchen aufgefüllt. Die Macrophagen werden zuvor aus einer Maus isoliert und in 10 ml HT-Medium resuspendiert (vgl. Abschnitt 3.1).

In diesen die Macrophagen enthaltenden Falcon-Röhrchen werden die Hybridzellen verdünnt, bis eine Zelldichte von (i) 270 Zellen/30 ml bzw. (ii) 90 Zellen/30 ml erreicht ist. Anschliessend werden diese Ansätze auf Costarplatten (Mikrotiterplatten mit 96 Vertiefungen) verteilt, wobei je 200 µl pro Vertiefung zugegeben werden. Dies entspricht einer Zellzahl von (i) 1,8 Zellen/Vertiefung bzw. (ii) 0,6 Zellen/Vertiefung. Pro Verdünnung benötigt man auf diese Weise 1,5 Mikrotiterplatten.

Nach 7 Tagen werden die einzelnen Vertiefungen mikroskopisch kontrolliert und die Vertiefungen die Zellklone enthalten erfasst. Diejenige Verdünnung, bei der ca. 50 % der Vertiefungen Zellklone enthalten wird für den ELISA-Test verwendet. Dies sollte in der Regel die Verdünnung mit 0,6 Zellen/Vertiefung sein. Nach ca. 7 - 10 Tagen werden die Ueberstände der positiven Vertiefungen (mit Klonen) in einem ELISA-Test auf die Anwesenheit von monoklonalen Antikörpern getestet und die positiven Klone auf Costarplatten (mit 24 Bohrungen) in RPMI 1640-Medium vermehrt. Aliquots dieser positiven Klone werden in flüssigem Stickstoff aufbewahrt.

Beispiel 6: Hybridoma-Screening (ELISA-Test)

Zunächst werden 100 µl einer Lösung von BSA-konjugiertem Hapten [2 µg/ml] in Natriumcarbonat-Puffer (50 mM, pH 9.6) in die einzelnen Vertiefungen einer Mikrotiterplatte gegeben und dieser Ansatz bei 4°C in einer feuchten Kammer über Nacht inkubiert. Anschliessend werden die Vertiefungen jeweils 5 x mit einem 0,1 % PBS-Tween Puffer gewaschen. Zur Blockierung der unbesetzten Bindungsstellen auf der Mikrotiterplatte werden 200 µl einer PBS-BSA Lösung (1 %) in jede Vertiefung gegeben. Dieser Ansatz wird 1 - 2 Stunden bei Raumtemperatur inkubiert und anschliessend mit einem 0,1 %igen PBS-Tween-Puffer gewaschen.

Man gibt dann zu jeder Vertiefung 200 µl des Hybridoma-Überstands, der im Verhältnis 1:2 mit PBS-Tween (0,1 %) verdünnt ist, hinzu und inkubiert den gesamten Ansatz für 2 Stunden bei Raumtemperatur. Anschliessend werden die Vertiefungen erneut 5 x mit einem 0,1 %igen PBS-Tween-Puffer gewaschen.

Es folgt die Inkubation mit Phosphatase-konjugiertem Ziegen anti-Maus Antikörper (Kirkegaard & Perry Lab.). Zunächst werden 100 µl eines über eine Affinitätschromatographie gereinigten Ziegen Antikörpers gegen Mäuse IgG, der 1 : 1500 in PBS-Tween (0,1 %) verdünnt vorliegt (Kirkegaard & Perry Laboratories) und mit alkalischer Phosphatase markiert ist, zu jeder Vertiefung hinzugegeben.

Die Inkubationszeit beträgt 1,5 Stunden bei Raumtemperatur. Anschliessend werden die einzelnen Vertiefungen erneut mit PBS-Tween (0,1 %) gewaschen (5 x).

Danach werden 150 µl einer substrathaltigen Lösung (1 mg/ml p-Nitrophenylphosphat) in jede Vertiefung zugegeben. Nach einer Inkubationszeit von 2 Stunden im Dunkeln erfolgt die spektroskopische Bestimmung bei 405 nm. Positive Hybridoma-Zellen, die einen spezifischen Antikörper sezernieren geben ein starkes positives Signal bei der gewählten Wellenlänge. Diese werden dann mit Hilfe des limitierenden Verdünnungsverfahrens [Godings (1980)] kloniert. Reine monoklonale Antikörper können aus der Ascitesflüssigkeit entsprechend vorbehandelter Mäuse gewonnen werden [Campbell AM, (1984)]

<u>Beispiel 7:</u> <u>Expansion der Hybridomazellen in der Maus</u>

Für die Anregung der Ascites-Produktion werden weibliche Balb/c Mäuse (20 - 25 mg) (Tierfarm Sisseln, CH) mit 0.3 ml Pristan Oel (Aldrich Chemical) vorbehandelt, das intraperitoneal injiziert wird. 1 bis 3 Wochen nach der Pristan Applikation erhalten die Mäuse eine zweite Injektion (0,2 ml Pristan-Oel, i.p.). Gleichzeitig mit dieser 2. Injektion erhalten die Tiere 2 x $10^6$ Hybridomazellen in 0,2 ml PBS.

Die aus dieser Behandlung resultierende Ascitesflüssigkeit wird gesammelt, bei 800 g zentrifugiert und bei einer Temperatur von -20°C aufbewahrt. Nach dem Auftauen wird die Ascitesflüssigkeit 1 Stunde bei 30'000 g zentrifugiert. Die oberste Schicht, die vorwiegend Lipide enthält, wird entfernt. Anschliessend wird die Proteinkonzentration bestimmt und auf einen Wert von 10 mg/ml eingestellt durch Zugabe von PBS.

Die Immunglobulin G Fraktion (IgG) wird durch tropfenweise Zugabe von 0,9 Volumenteilen einer gesättigten Ammoniumsulfatlösung bei 0°C präzipitiert. Nach 1 Stunde wird die IgG-Fraktion pelletiert durch einstündige Zentrifugation bei 22'000 g. Das Pellet wird anschliessend in 20 mM-Tris-HCl Puffer, pH 7,9, der 50 mM NaCl enthält, aufgelöst und gegen den gleichen Puffer über Nacht bei 4°C dialysiert. Die weitere Aufarbeitung der IgG Fraktion geschieht mit Hilfe einer Anionen-Austauschchromatographie an einer DE-52 Diethylaminoethylcellulose (Whatman) Säule. Die Probe wird 1:2 (v/v) mit 20 mM Tris-HCl, pH 7,9 verdünnt, bis eine NaCl Endkonzentration von 25 mM erreicht ist und 10 mg Protein/ml Gel werden auf die Säule aufgetragen. Die Elution wird erreicht durch Erhöhung der NaCl-Konzentration von 25 mM auf 200 mM (linearer Gradient). Im allgemeinen erfolgt die Elution monoklonaler Antikörper im Bereich von 80 mM NaCl.

Die Fraktionen werden über Nacht bei einer Temperatur von 4°C gegen PBS dialysiert und bei -70°C aufbewahrt. Der Reinheitsgrad wird mit Hilfe einer Natriumdodecylsulphat Polyacrylamid-Gelelectrophorese (SDS-PAGE) bestimmt, sowie durch isoelektrische Fokussierung.

Im vorliegenden Fall liegt der Reinheitsgrad bei >90 %.

<u>Beispiel 8:</u> <u>Nachweis einer Verbindung der Formel (II)</u>

Der Nachweis der Verbindung gemäss Formel (II) kann mit Hilfe eines zweistufigen, kompetitiven ELISA-Tests durchgeführt werden.

BSA-konjugiertes Hapten, hergestellt gemäss Beispiel 1.2.1 wird zunächst in einem 50 mM Natriumcarbonatpuffer (pH 9,6) (200 ng BSA-konjugiertes Hapten/100 µl 50 mM Natriumcarbonat-Puffer) an Mikrotiterplatten (z.B. Dynatech, Typ M 129A) adsorbiert und über Nacht bei einer Temperatur von 4°C inkubiert.

Die Platten werden anschliessend 5 x mit PBS-Puffer gewaschen, der mit 0,1 % (v/v) Polysorbat 20 (Tween 20®) angereichert ist (PBS-Tween).

Die restlichen freien Bindungsstellen des festen Trägermaterials werden dann durch Zugabe von PBS-BSA in Form einer 1 % (w/v) Lösung blockiert. Nach zweistündiger Inkubation bei 22° C werden die Platten erneut mit PBS-Tween (0.1 %) gewaschen.

50 µl des Überstandes der zuvor klonierten Hybridomazellen (in einer Verdünnung von 1:1000 bis 1:2000) oder aber 50 µl der zuvor gereinigten monoklonalen Antikörper (40 -120 ng/ml) werden a) mit 950 µl einer Standardlösung, die einen steigenden Anteil an Antigen [Verbindung der Formel (II)] bzw. Antigen-Analogen enthält, b) mit Antigen-haltigen Wasserproben oder c) Antigen-haltigen Bodenextrakten inkubiert. [Alle Verdünnungen werden in PBS-Tween (0.1 %) vorgenommen].

Nach einer Inkubationszeit von 1 Stunde bei Raumtemperatur (22° C) werden 200 µl des Antigen/Antikörper Gemisches zu jeder Vertiefung der Mikrotiterplatte zugegeben und der gesamte Ansatz wird für eine weitere Stunde inkubiert. Die Vertiefungen werden anschliessend 5 x mit PBS-Tween (0.1 %) gewaschen und mit 100 µl/Vertiefung Ziegen-anti-Maus IgG Antikörper, der konjugiert an alkalische Phosphatase vorliegt (Verdünnung 1:1500), beschickt und für einen Zeitraum von 1,5 Stunden inkubiert.

Nach erneutem Waschen werden 150 µl/Vertiefung des in 1 mg/ml Diethanolaminpuffer (1 mM, pH 9,8, angereichert mit 0,5 mM $MgCl_2$ x $6H_2O$) gelösten Substrates p-Nitrophenylphosphat zu den Vertiefungen zugegeben. Nach einer Inkubationszeit von 2 Stunden bei einer Temperatur von 22° C lässt sich ein Farbumschlag beobachten, der proportional ist zur Menge an Antikörper, der mit dem an die Festphase gebundenen Antigen reagiert hat. Die Intensität der eingetretenen Farbreaktion wird bei einer Wellenlänge von 405 nm bestimmt. Die Verdünnungen der einzelnen Proben werden so gewählt, dass man ohne Zugabe eines Inhibitors (Bo) Absorptionswerte in einem Bereich zwischen 0.3 und 0.5 erhält. Für die Kontrollen (ohne Antikörper) ergeben sich Werte von $A_{405} \leq 0.01$. Alle Proben werden in dreifacher Ausfertigung bestimmt.

Zur Ermittlung der in einer Probe enthaltenen Menge an Antigen [Verbindung der Formel (II)] wird zunächst eine Eichkurve erstellt, wobei B/Bo x 100 gegen die Konzentration an Inhibitor aufgetragen wird. (Bo bedeutet Absorptionsfähigkeit gemessen ohne Zugabe eines Antigen-Inhibitors zum Antikörper, und B Absorptionsfähigkeit bei Zugabe verschieden konzentrierter Antigen-Inhibitoren) Der $I_{50}$-Wert gibt diejenige Konzentration des Antigens an, bei der die Antikörperbindung an die Festphase zu 50 % gehemmt wird. Der $I_{50}$-Wert wird mit Hilfe eines auf die vorliegenden Verhältnisse speziell angepassten ENZFITTER (Leatherbarrow, Elsevier-Biosoft) Kurvenkalkulationsprogramms bestimmt, basierend auf einer 4 Parameter umfassenden logistischen Kurve (Raab GM, 1983). Auch die quantitative Antigenbestimmung in Boden- oder Wasserproben im Rahmen des ELISA wird mit Hilfe des ENZFITTER-Programms durchgeführt, wobei die Anpassung der Kurve auf Standards basiert, die auf jeder Mikrotiterplatte mitlaufen.

Beispiel 8.1: Analyse von Bodenproben

Aliquots (2 g) standardisierter Bodenproben unterschiedlicher Herkunft werden gemäss der im folgenden beschriebenen Verfahren extrahiert:

Methode (A) [nach Iwanzik und Egli (1989)]: Ein 100 g Probe wird 2 Stunden in einem Extraktor durch Ausschütteln mit 300 ml eines 2:1 Gemisches (v/v) aus Methanol und einem wässrigen Phosphatpuffer (PB) [pH 7.0, Gesamt-Phosphatkonzentration 0.07 M] extrahiert. Nach Filtration und Ansäuern mit Phosphorsäure wird die Sulfonylharnstoff Verbindung 3 x mit 75 ml $CH_2Cl_2$ re-extrahiert. Nach Abdampfen des Lösungsmittels wird die Probe in 10 ml PB-Puffer gelöst und über eine Filtration gereinigt.

Methode (B): Die Extraktion nach Methode (A) wird mit zwei weiteren Proben wiederholt und die letzte organische Phase durch Ausschütteln mit einer wässrigen Hydrogencarbonat-Lösung (5%) weiter gereinigt [Iwanzik und Egli (1989)]. Nach Zugabe von Tetrabuthylammoniumhydrogencarbonat wird die Sulfonylharnstoff Verbindung in Dichlormethan-n-hexan (80:20) re-extrahiert. Nach Aodampfen der organischen Phase wird die Sulfonylharnstoff Verbindung in 10 ml PB-puffer aufgenommen. Bevor die Probe im ELISA Test verwendet wird, wird sie in PBS-Tween 0.1 % im Verhältnis 1:20 oder 1:40 verdünnt.

Methode (C): In diesem Fall erfolgt das Ausschütteln mit Tetrabythylammoniumhydroxid direkt mit dem Methanol/PB Extrakt. Die wässrige Phase wird anschliessend auf ein flüssig-flüssig 'Partitioning Cartridge' [ClinElut® #1010, Analytichem International, Harbor City, CA] überführt und mit 30 ml n-Hexan gewaschen. Die Sulfonylharnstoff Verbindung wird mit Dichlormethan/n-Hexan (60:40) eluiert. Die organische Phase wird verdampft und der zurückbleibende Rest in PBS aufgenommen.

Beispiel 8.2: Analyse von Wasserproben

Für den kompetitiven ELISA werden 100 µl eines 10fach konzentrierten PBS-Tween Puffers zu 850 µl einer Wasserprobe zugegeben. Dieser Ansatz wird schliesslich mit 50 µl des Antikörpers inkubiert.

Beispiel 9: Immunopurification

Die gemäss Beispiel 7 hergestellten monoklonalen Antikörper werden an einen festen Träger immobilisiert. Dabei handelt es sich vorzugsweise um ein mit Cyanbromid [CN-Br] aktiviertes SEPHAROSE 4B® Gel. Dieses wird anschliessend in eine im Rahmen von Reinigungsverfahren routinemässig verwendeten Minisäulen gepackt.

Vor dem Aufbringen der zu analysierenden Bodenprobe wird die Säule durch Waschen mit 5 bis 10 ml PBS Puffer [pH 7.4] konditioniert.

Der wässrige Bodenprobenextrakt wird in 50 ml Pufferlösung aufgenommen und tropfenweise auf die Säule appliziert. Anschliessend wird die Säule ein oder mehrmals mit 20 ml einer Waschlösung bestehend aus Wasser oder einem 9:1 Gemisch von Wasser und Acetonitril gewaschen.

Die Elution des Analyten erfolgt durch Zugabe einer Acetonitiril-Elutionslösung, die vorzugsweise mit 5% einer phosphorigen Säure angereichert wird. Dabei gilt, dass die Effiziens des Elutionsprozesses mit abnehmender Durchflussrate des Eluats zunimmt. Optimal ist eine Durchflussrate von < 0.5 %.

## II. ERGEBNISSE

1) Herstellung monokonaler Antikörper

(a) Ausgehend von 4 Fusionsereignissen unter Verwendung von Mäusen, die mit dem gemäss Beispiel 1.2.1 hergestellten KLH-Konjugat immunisiert wurden, erhält man ein Hybridoma, welches einen monoklonalen Antikörper mit hoher Affinität gegenüber einer Verbindung der Formel (II) produziert [MAb 4197-70-12]. Die Fusionseffizienz liegt bei ca. 90 %.

Die ermittelten Kreuzreaktivitäten dieses MAbs gegenüber den in Tabelle 1 wiedergegebenen Verbindungen liegt unterhalb von 0.1 %. Besonders bemerkenswert ist dabei, das besagter MAb keine Kreuzreaktivität mit dem strukturell sehr nahe verwandten Triasulfuron aufweist.

Die untere Nachweisgrenze für die Zielverbindung [Verbindung der Formel (II)] unter Verwendung des erfindungsgemässen MAb (in Puffer) liegt im Bereich von 0.1 ng/ml Puffer. Der entsprechende $I_{50}$-Wert für MAb 4197-70-12 beträgt 0.8 ng/ml

## III. HINTERLEGUNG

Die im Rahmen der vorliegenden Erfindung hergestellte und verwendete Hybridoma-Zellinie wurde am 06. Dezember 1991 bei der als Internationale Hinterlegungsstelle anerkannten 'European Collection of Animal Cell Cultures' (ECACC) in Salisbury, UK, entsprechend den Anforderungen und Bestimmungen des Budapester Vertrages für die Internationale Anerkennung der Hinterlegung von Mikroorganismen zum Zwecke der Patentierung, unter der Hinterlegungsnummern ECACC 911 20619 hinterlegt. Eine Erklärung zur Lebensfähigkeit der hinterlegten Proben wird durch die besagte Internationale Hinterlegungsstelle ausgefertigt.

| Zellinie | Hinterlegungsdatum | Hinterlegungsnummer | Datum der Lebensfähigkeitsbescheinigung |
|---|---|---|---|
| Hybridoma Klon 4197-70-12 | 06.12.1991 | 911 20619 | 06.12.1991 |

## IV. MEDIEN UND PUFFER

(A) RPMI 1640-Medium

RPMI 1640 (Seromed) mit folgenden Zusätzen:

| | |
|---|---|
| Kälberserum | 15 % |
| L-Glutamin | 4 mM |
| Gentamycin | 0.01% |
| Natrium-Pyruvat | 1 mM |
| 2-Mercaptoethanol | 50 µM |
| Insulin | 5 µM |
| Transferrin | 5 µM |
| Selen (ITS) | 5 µM |

(B) HAT-Medium

1 Liter RPMI 1640 Medium mit 20 ml Zusatz von HAT conc. (50 x) von Boehringer, das die folgende Zusammensetzung aufweist:

| | |
|---|---|
| Hypoxanthin 680 | 5.0 mg/l |
| Aminopterin | 8.8mg/l |
| Thymidin | 193,8 mg/l |

(C) HT-Medium

1 Liter RPMI 1640 Medium mit 20 ml Zusatz von HT conc. (50 x) von Boehringer, das die folgende Zusammensetzung aufweist:

| | |
|---|---|
| Hypoxanthin 680 | 5.0 mg/l |
| Thymidin | 193,8 mg/l |

(D) BSS-Puffer [Earle's Salzlösung, ohne Ca und Mg, pH 7.4]

| | |
|---|---|
| KCl | 7.3 mM |
| NaCl | 116.0 mM |
| $NaHCO_3$ | 26.0 mM |
| $NaH_2PO_4 \cdot 2H_2O$ | 1.0 mM |
| Glucose | 5.5 mM |
| Phenolrot | 48.0 µM |

1% Zusatz (v/v) einer Penicillin/Streptomycin-Lösung (Seromed) [10'000E Penicillin, 10 mg/ml Streptomycin]

(E) Natriumcarbonat-Puffer [pH 9.6]

| | |
|---|---|
| $Na_2CO_3$ | 477.0 mg |
| $NaHCO_3$ | 879.0 mg |
| $NaN_3$ | 1.8 mg |
| ad 300 ml $H_2O$ | |

(F) PBS-Puffer [pH 7.0]

| | |
|---|---|
| NaCl | 8.5 g |
| $Na_2HPO_4 \cdot 2H_2O$ | 1.28 g |
| $NaH_2PO_4 \cdot 2H_2O$ | 0.436 g |
| ad 1000 ml $H_2O$ | |

(G) PBS-TWEEN-20 [0.1%]

1 ml Tween-20® (Serva) + 1000 ml PBS

(H) PBS-BSA [1%]

| | |
|---|---|
| BSA | 5.0 g |
| $NaN_3$ (0.5 M) | 3.0 ml |
| ad 500 ml PBS | |

(I) Substrat-Puffer [Diethanolamin-Puffer, pH 9.8]

| | |
|---|---|
| Diethanolamin | 97.0 ml |
| $NaN_3$ (0.5 M) | 6.0 ml |
| $MgCl_2 \cdot 6H_2O$ | 100.0 mg |
| ad 1000 ml $H_2O$, Einstellen des pH-Wertes auf pH 9.8 mit HCl conc. | |

Herstellung des Substrates: Unmittelbar vor Gebrauch wird eine Substrattablette (= 5 mg) des p-Nitrophenylphosphat-Substrates (Sigma 104) in 5 ml Substratpuffer gelöst.

## V. LITERATURVERZEICHNIS


**Ahmad** I und Crawford G, J. Agric. Food Chem., 38: 138-141, 1990

**Campbell** AM, "Monoclonal Antibody Technology", in: Laboratory Techniques in Biochemistry and Molecular Biology; Burdon, RH; Knippenberg PH (Eds.); Elsevier: Amsterdam, 1984; Vol. 13, pp. 120-184

**DeLuca,** "Immunofluorescence Analysis", in: Antibody As a Tool, Marchalonis et al, John Wiley & Sons, Ltd., pp 189-231 (1982)

**Ercegovich** CD et al, J. Agric. Food Chem., 29: 559-563, 1981

**Feng** *et al*, J. Agric. Food Chem., 38: 159-163, 1990
**Fleeker** J, J. Assoc. Off. Anal. Chem., 70: 874-878, 1987
**Hargrave** HS und Merkle MG, Weed Sci., 19: 1971
**Iwanzik** W *et al*, Z. PflKrankh. PflSchutz, Suppl. XI: 301-310, 1988
**Kawamura** H, Berzojsky JA, J. Immunol., 136: 58, 1986
**Kelley** M et al, J. Agric. Food Chem., 33: 962-965, 1985
**Köhler** G, Milstein, Nature, 256: 495-497, 1975
**Kulkarni** NP et al, Cancer Res., 41: 2700-2706, 1981
**Littlefield** JW, Science, 145: 709, 1964
**Newsome** WH, J. Agric. Food Chem., 33: 528-530, 1985
**Raab** GM, Clin. Chem., 29: 1757-1761, 1983
**Schlaeppi** J-M *et al*, J. Agric. Food Chem., 37: 1532-1538, 1989
**Shulman** M et al, Nature, 276: 269-270, 1978
**Stocker** JW *et al*, Hoffmann-La Roche Research Disclosure, 21713: 155-157, 1982
**van Rensburg** E, Analyst, 110: 733., 1985
**Wie** SI, Hammock BD, J. Agric. Food Chem., 30: 949-957, 1982
**Zahnow** EW, J. Agric. Food Chem., 30: 854-857, 1982

Patentliteratur

**EP-0,120,814**

## VI. TABELLEN

Tabelle 1

| Kreuzreaktivitäten verschiedener Analoger der Verbindung gemäss Formel (II) | | |
|---|---|---|
| Compound | MAb 4197-70-12 | |
| | (a) $I_{50}$ (ng/ml) | (b) Kreuzreaktivität (%) |
| Verbindung d. Formel (II) | 0.8 | 100 |
| A | >1000 | <0.1 |
| B | >1000 | <0.1 |
| C | >1000 | <0.1 |
| D | >1000 | <0.1 |

a) Inhibitorkonzentration, welche das ELISA Signal um 50 % im Vergleich zur Kontrolle reduziert.
b) (Antigenkonz. für 50 % Hemmung / Konzentration des Antigenanalogen für 50 % Hemmung) x 100.

Die Analogen der Verbindung gemäss Formel (II) A bis D sind im folgenden anhand ihrer Strukturformeln wiedergegeben.

(I) $A\text{-}SO_2\text{-}NH\text{-}CO\text{-}NH\text{-}B$

A = 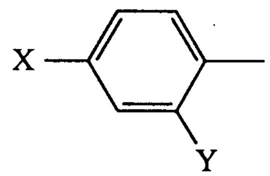 B = 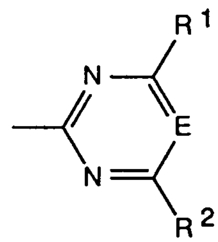

| | Y | E | $R^1$ | $R^2$ | X |
|---|---|---|---|---|---|
| (A) | $-OCH_2CH_2Cl$ | -N | $-OCH_3$ | $-CH_3$ | -OH |
| (B) Triasulfuron | $-OCH_2CH_2Cl$ | -N | $-OCH_3$ | $-CH_3$ | -H |
| (C) Chinosulfuron | $-OCH_2CH_2OCH_3$ | -N | $-OCH_3$ | $-OCH_3$ | -H |
| (D) Primisulfuron | $-COOCH_3$ | -C | $-OCHF_2$ | $-OCHF_2$ | -H |

## Patentansprüche

1. Verfahren zur Herstellung eines monoklonalen Antikörpers mit hoher Spezifität und Affinität gegenüber einer oder mehreren Verbindungen der Formel (A),

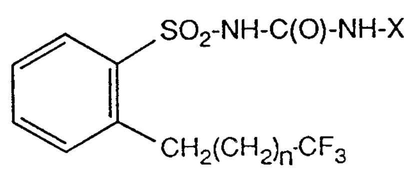

(A)

worin

X für einen über Kohlenstoff gebundenen, ein- oder zweifach substituierten 6-Ring-Heterozyklus mit ein bis 3 Stickstoffatomen steht; und
n eine ganze Zahl von 0 bis 4 repräsentiert, dadurch gekennzeichnet, dass man

(a) eine Kupplungskomponente, die aus einem Fragment besteht, das insbesondere den Sulfonamidteil der Verbindung der Formel A umfasst mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und

(f) besagte Hybridomazellen zur Herstellung monoklonaler Antikörper *in vitro* oder *in vivo* kultiviert.

2. Verfahren gemäss Anspruch 1, dadurch gekennzeichnet, dass X für ein- bis zweifach substituiertes Triazin oder Pyrimidin steht.

3. Verfahren gemäss Anspruch 2 zur Herstellung eines monoklonalen Antikörpers mit hoher Spezifität und Affinität gegenüber einer oder mehreren Verbindungen der Formel (I),

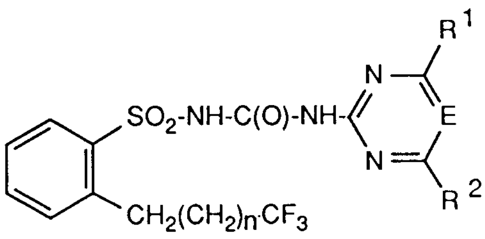

(I)

worin

$R^1$ und $R^2$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder -$NR^3R^4$ bedeuten, worin
$R^3$ und $R^4$ unabhängig voneinander für Wasserstoff oder $C_1$-$C_4$-Alkyl stehen;
E Stickstoff oder die Methinbrücke bedeuten; und
n eine ganze Zahl von 0 bis 4 repräsentiert, dadurch gekennzeichnet, dass man

(a) eine Kupplungskomponente, die aus einem Fragment besteht, das insbesondere den Sulfonamidteil der Verbindung der Formel (I) umfaßt und dieses, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert.

4. Verfahren zur Herstellung eines monoklonalen Antikörpers mit hoher Spezifität und Affinität gegenüber einer Verbindung der Formel (II),

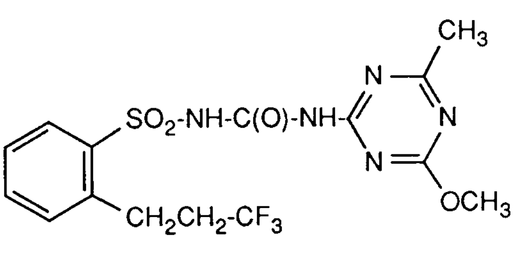

(II)

dadurch gekennzeichnet, dass man

(a) eine Kupplungskomponente, die aus einem Fragment besteht, das insbesondere den Sulfonamidteil der Verbindung der Formel (II) umfaßt und dieses mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;

(e) das entstehende Fusionsprodukt isoliert und nach Selektion diejenigen Hybridomazellen kloniert, die den gewünschten Antikörper produzieren und

(f) besagte Hybridomazellen zur Herstellung monoklonaler Antikörper *in vitro* oder *in vivo* kultiviert.

**5.** Verfahren gemäss einem der Ansprüche 1 bis 4, dadurch gekennzeichnet, dass es sich bei besagter Kupplungskomponente um eine Verbindung gemäss Formel (III) handelt,,

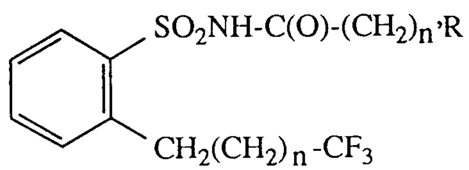

(III)

worin

R für COOH, $NH_2$ oder SH steht; und
n' eine ganze Zahl von 1 bis 10; und
n eine ganze Zahl von 0 bis 4 repräsentiert.

**6.** Verfahren gemäss einem der Ansprüche 1 bis 4, dadurch gekennzeichnet, dass man als Carriermolekül makromolekulare Verbindungen verwendet, die frei zugängliche reaktive Gruppen für die Kupplungsreaktion mit der Sulfonylharnstoff-Kupplungskomponente aufweisen und die in der Lage sind, der Sulfonylharnstoff-Komponente eine immunogene Potenz zu verleihen.

**7.** Verfahren gemäss einem der Ansprüche 1 bis 4, dadurch gekennzeichnet, dass die Konjugation der Sulfonylharnstoff-Kupplungskomponente an das Carriermolekül direkt oder aber über ein Brückenglied (Spacer) erfolgt, das eine oder mehrere reaktive Gruppen besitzt, die in der Lage sind mit den reaktiven Gruppen des Carriermoleküls in Wechselwirkung zu treten.

**8.** Verfahren gemäss einem der Ansprüche 1 bis 4, dadurch gekennzeichnet, dass die Immunisierung der Donor-Tiere durch ein- bis mehrmalige Applikation eines Konjugates, bestehend aus Kupplungskomponente und Carrier-Molekül erfolgt.

**9.** Verfahren gemäss einem der Ansprüche 1 bis 4, dadurch gekennzeichnet, dass man für die Selektion fusionierte Hybridzellen das HAT-Selektionsmedium verwendet.

**10.** Verfahren gemäss einem der Ansprüche 1 bis 4, dadurch gekennzeichnet, dass man positive, monoklonale Antikörper produzierende Hybridzellkulturen mit Hilfe des limitierenden Verdünnungsverfahrens vereinzelt und anschliessend die so gewonnene Reinkultur in einem geeigneten Kultivierungsmedium kloniert.

**11.** Verfahren gemäss einem der Ansprüche 1 bis 4, dadurch gekennzeichnet, dass man die gemäss Anspruch 10 klonierten Hybridoma-Zellklone mit Hilfe eines Immunoassays auf die Bildung geeigneter monoklonaler Antikörper hin untersucht.

**12.** Monoklonaler Antikörper, der aus der Hybridomazellinie mit den kennzeichnenden Charakteristika von ECACC 911 20619 erhältlich ist, sowie Derivate von besagtem monoklonalem Antikörper.

**13.** Monoklonaler Antikörper, der eine hohe Spezifität und Affinität gegenüber einer oder mehreren Verbindungen der Formel (A),

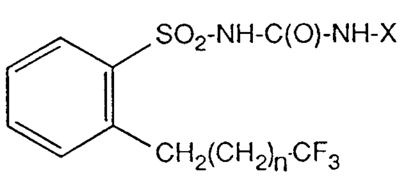

(A)

worin

X für einen über Kohlenstoff gebundenen, ein- oder zweifach substituierten 6-Ring-Heterozyklus mit ein bis 3 Stickstoffatomen steht; und
n eine ganze Zahl von 0 bis 4 repräsentiert, aufweist und herstellbar ist durch ein Verfahren, das dadurch gekennzeichnet ist, dass man

(a) eine Kupplungskomponente, die aus einem Fragment besteht, das insbesondere den Sulfonamidteil der Verbindung der Formel (A) umfaßt mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert;
(e) das entstehende Fusionsprodukt isoliert und nach Selektion diejenigen Hybridomazellen kloniert, die den gewünschten Antikörper produzieren und
(f) besagte Hybridomazellen zur Herstellung monoklonaler Antikörper *in vitro* oder *in vivo* kultiviert.

**14.** Monoklonaler Antikörper gemäss Anspruch 13, dadurch gekennzeichnet, dass er eine hohe Spezifität und Affinität gegenüber einer oder mehreren Verbindungen der Formel (I),

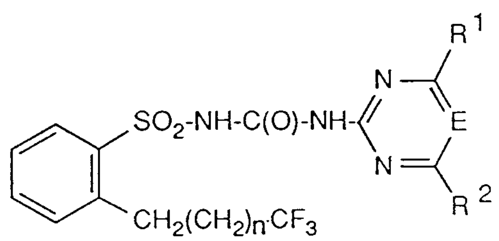

(I)

worin

$R^1$ und $R^2$ unabhängig voneinander Wasserstoff, Halogen, $C_1$-$C_4$-Alkyl, $C_1$-$C_4$-Halogenalkyl, $C_2$-$C_4$-Halogenalkoxy, $C_1$-$C_4$-Alkylthio, $C_1$-$C_4$-Halogenalkylthio, $C_2$-$C_4$-Alkoxyalkyl, Methoxy, Aethoxy oder -$NR^3R^4$ bedeuten, worin
$R^3$ und $R^4$ unabhängig voneinander für Wasserstoff oder $C_1$-$C_4$-Alkyl stehen;
E Stickstoff oder die Methinbrücke bedeuten; und
n eine ganze Zahl von 0 bis 4 repräsentiert, aufweist.

**15.** Monoklonaler Antikörper gemäss Anspruch 14, dadurch gekennzeichnet, dass er eine hohe Spezifität und Affinität gegenüber einer Verbindung der Formel (II),

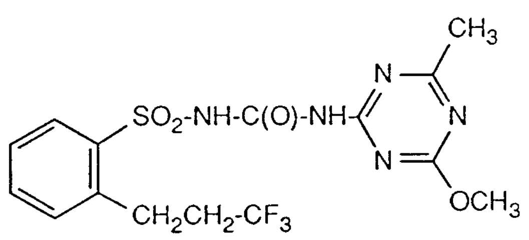

(II)

aufweist.

**16.** Monoklonaler Antikörper gemäss einem der Ansprüche 13 bis 15, dadurch gekennzeichnet, dass es sich in Schritt (a) bei besagter Kupplungskomponente um eine Verbindung gemäss Formel (III) handelt,,

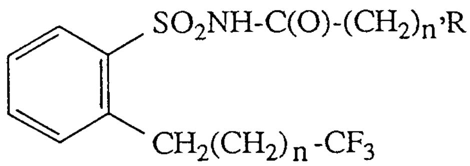

(III)

worin

R für COOH, $NH_2$ oder SH steht; und
n' eine ganze Zahl von 1 bis 10; und
n eine ganze Zahl von 0 bis 4 repräsentiert.

**17.** Hybridomazellinie ECACC 911 20619 sowie Klone und Subklone davon.

**18.** Hybridomazellinie, die einen monoklonalen Antikörper produziert, der eine hohe Spezifität und Affinität gegenüber einer oder mehreren Verbindungen der Formel (A),

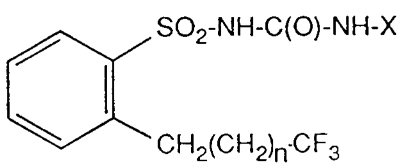

(A)

worin

X für einen über Kohlenstoff gebundenen, ein- oder zweifach substituierten 6-Ring-Heterozyklus mit ein bis 3 Stickstoffatomen steht; und
n eine ganze Zahl von 0 bis 4 repräsentiert, aufweist und herstellbar ist durch ein Verfahren, das dadurch gekennzeichnet ist, dass man

(a) eine Kupplungskomponente, die aus einem Fragment besteht, das insbesondere den Sulfonamidteil der Verbindung der Formel (A) umfaßt, mit einem geeigneten, hochmolekularen Carrier-Molekül konjugiert;
(b) mit dem gemäss (a) hergestellten Konjugat ein Donor-Tier immunisiert;
(c) immunkompetente B-Zellen aus dem immunisierten Donor-Tier isoliert;
(d) besagte immunkompetente B-Zellen mit zu einer kontinuierlichen Zellteilung befähigten Tumorzellen fusioniert; und
(e) das entstehende Fusionsprodukt isoliert und nach Selektion diejenigen Hybridomazellen kloniert, die den gewünschten Antikörper produzieren.

**19.** Mutanten und Varianten der Hybridomazelline gemäss einem der Ansprüche 17 oder 18, die spontan entstehen oder aber mit Hilfe bekannter Mutationsverfahren herstellbar sind.

**20.** Verfahren zum immunologischen Nachweis von Sulfonylharnstoff-Herbiziden der allgemeinen Formel (I), dadurch gekennzeichnet, dass man einen monoklonalen Antikörper gemäss einem der Ansprüche 13 oder 14 in einen, der bekannten Immunoassays anwendet.

**21.** Verfahren zum immunologischen Nachweis einer Verbindung gemäss Formel (II), dadurch gekennzeichnet, dass man einen monoklonalen Antikörper gemäss einem der Ansprüche 12 oder 15 in einem der bekannten Immunoassays anwendet.

**22.** Verfahren zum immunologischen Nachweis einer Verbindung der Formel (II) gemäss Anspruch 21, dadurch gekennzeichnet, dass man einen monoklonalen Antikörper verwendet, der aus einer Hybridomazellinie erhältlich ist, welche die kennzeichnenden Charakteristika von ECACC 911 20619 besitzt oder aus Klonen oder Subklonen

davon.

**23.** Verfahren gemäss einem der Ansprüche 20 bis 22, dadurch gekennzeichnet, dass es sich bei besagtem Immunoassay um einen kompetitiven Immunassay handelt.

**24.** Mittel zum immunologischen Nachweis von Sulfonyulharnstoff-Herbiziden der allgemeinen Formel (I) in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen monoklonalen Antikörper gemäss einem der Ansprüche 13 oder 14 als Reagenz enthält.

**25.** Mittel zum immunologischen Nachweis einer Verbindung der Formel (II) in Form eines gebrauchsfertigen Test-Kits, dadurch gekennzeichnet, dass besagter Test-Kit neben den üblicherweise verwendeten Trägermaterialien, Reagentien und sonstigen Zusätzen mindestens einen monoklonalen Antikörper gemäss einem der Ansprüche 12 oder 15 als Reagenz enthält.

**26.** Mittel zum immunologischen Nachweis einer Verbindung der Formel (II) in Form eines gebrauchsfertigen Test-Kits gemäss Anspruch 25, dadurch gekennzeichnet, dass besagter Test-Kit neben den üblicherweise verwendeten Trägermaterialien, Reagentien und sonstigen Zusätzen mindestens einen monoklonalen Antikörper enthält, der aus einer Hybridomazellinie, welche die kennzeichnenden Charakteristika von ECACC 911 20619 besitzt oder aus Klonen oder Subklonen davon, erhältlich ist.

**27.** Kupplungskomponente der Formel (III)

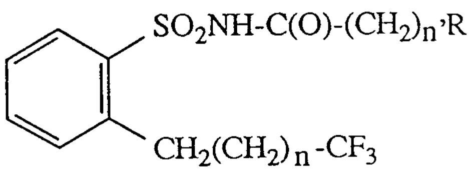

(III)

worin

R für COOH, $NH_2$ oder SH steht; und
n' eine ganze Zahl von 1 bis 10; und
n eine ganze Zahl von 0 bis 4 repräsentiert

**28.** Verwendung eines monoklonalen Antikörpers gemäss einem der Ansprüche 13 oder 14 in einem 'Immunopurification' Verfahren, dadurch gekennzeichnet, dass man

(a) besagten Antikörper an einen festen Träger immobilisiert;
(b) besagten Träger in eine Minisäule plaziert;
(c) eine Pufferlösung enthaltend die zu reinigende oder zu analysierende Probe auf die Säule appliziert;
(d) gegebenfalls einen oder mehrere Wasch-Schritte zwischenschaltet; und
(e) den Analyten mit einer geeigneten Elutionslösung von der Säule eluiert.

**29.** Verwendung eines monoklonalen Antikörpers gemäss einem der Ansprüche 12 oder 15 in einem 'Immunopurification' Verfahren, dadurch gekennzeichnet, dass man

(a) besagten Antikörper an einen festen Träger immobilisiert;
(b) besagten Träger in eine Minisäule plaziert;
(c) eine Pufferlösung enthaltend die zu reinigende oder zu analysierende Probe auf die Säule appliziert;
(d) gegebenfalls einen oder mehrere Wasch-Schritte zwischenschaltet; und
(e) den Analyten mit einer geeigneten Elutionslösung von der Säule eluiert.

**Claims**

**1.** A method for the production of a monoclonal antibody having a high degree of specificity and affinity towards one or more compounds of formula (A)

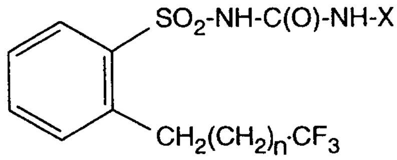

(A),

wherein

X is a mono- or di-substituted 6-membered heterocycle having from one to three nitrogen atoms and bonded via carbon; and
n is an integer from 0 to 4, wherein

(a) a linking component that consists of a fragment that comprises especially the sulfonamide moiety of the compound of formula (A) is conjugated with a suitable high molecular weight carrier molecule;
(b) a donor animal is immunised with the conjugate prepared in accordance with (a);
(c) immunocompetent B cells are isolated from the immunised donor animal;
(d) the said immunocompetent B cells are fused with tumour cells capable of continuous cell division;
(e) the resulting fusion product is isolated and after selection the hybridoma cells that produce the desired antibody are cloned, and
(f) the said hybridoma cells are cultured *in vitro* or *in vivo* to produce monoclonal antibodies.

**2.** A method according to claim 1 wherein X is mono- or di-substituted triazine or pyrimidine.

**3.** A method according to claim 2 for the production of a monoclonal antibody having a high degree of specificity and affinity towards one or more compounds of formula (I)

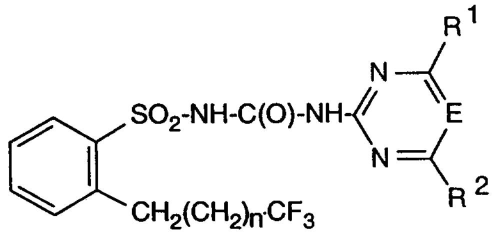

(I),

wherein

$R^1$ and $R^2$ are each independently of the other hydrogen, halogen, $C_1$-$C_4$alkyl, $C_1$-$C_4$haloalkyl, $C_2$-$C_4$haloalkoxy, $C_1$-$C_4$alkylthio, $C_1$-$C_4$haloalkylthio, $C_2$-$C_4$alkoxyalkyl, methoxy, ethoxy or $-NR^3R^4$, wherein $R^3$ and $R^4$ are each independently of the other hydrogen or $C_1$-$C_4$alkyl;
E is nitrogen or a methine bridge; and
n is an integer from 0 to 4, wherein

(a) a linking component that consists of a fragment that comprises especially the sulfonamide moiety of the compound of formula (I) is conjugated with a suitable high molecular weight carrier molecule;
(b) a donor animal is immunised with the conjugate prepared in accordance with (a);
(c) immunocompetent B cells are isolated from the immunised donor animal;
(d) the said immunocompetent B cells are fused with tumour cells capable of continuous cell division;
(e) the resulting fusion product is isolated and after selection the hybridoma cells that produce the desired antibody are cloned, and
(f) the said hybridoma cells are cultured *in vitro* or *in vivo* to produce monoclonal antibodies.

**4.** A method for the production of a monoclonal antibody having a high degree of specificity and affinity towards a compound of formula (II)

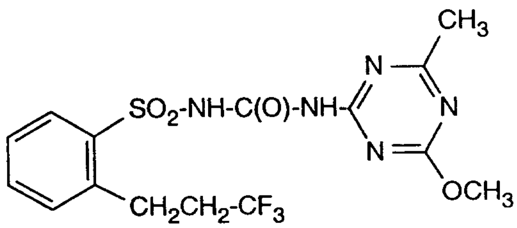

(II),

wherein

(a) a linking component that consists of a fragment that comprises especially the sulfonamide moiety of the compound of formula (II) is conjugated with a suitable high molecular weight carrier molecule;
(b) a donor animal is immunised with the conjugate prepared in accordance with (a);
(c) immunocompetent B cells are isolated from the immunised donor animal;
(d) the said immunocompetent B cells are fused with tumour cells capable of continuous cell division;
(e) the resulting fusion product is isolated and after selection the hybridoma cells that produce the desired antibody are cloned, and
(f) the said hybridoma cells are cultured *in vitro* or *in vivo* to produce monoclonal antibodies.

**5.** A method according to any one of claims 1 to 4 wherein the said linking component is a compound of formula (III)

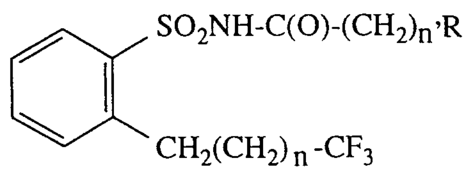

(III),

wherein

R is COOH, $NH_2$ or SH; and
n' is an integer from 1 to 10; and
n is an integer from 0 to 4.

**6.** A method according to any one of claims 1 to 4, wherein there are used as carrier molecules macromolecular compounds that have freely accessible reactive groups for the linking reaction with the sulfonylurea linking component and that are capable of imparting an immunogenic potential to the sulfonylurea component.

**7.** A method according to any one of claims 1 to 4, wherein the conjugation of the sulfonylurea linking component to the carrier molecule is effected directly or via a bridge member (spacer) having one or more reactive groups that are capable of interacting with the reactive groups of the carrier molecule.

**8.** A method according to any one of claims 1 to 4, wherein the immunisation of the donor animals is effected by means of one or more administrations of a conjugate consisting of linking component and carrier molecule.

**9.** A method according to any one of claims 1 to 4, wherein the HAT selection medium is used for the selection of fused hybrid cells.

**10.** A method according to any one of claims 1 to 4, wherein positive, monoclonal-antibody-producing hybrid cell cultures are isolated by means of the limiting dilution method and the pure culture thus obtained is then cloned in a suitable culture medium.

**11.** A method according to any one of claims 1 to 4, wherein the hybridoma cell clones cloned in accordance with claim 10 are examined by means of an immunoassay for the formation of suitable monoclonal antibodies.

**12.** A monoclonal antibody that is obtainable from the hybridoma cell line having the distinguishing characteristics of

ECACC 911 20619, or a derivative of the said monoclonal antibody.

**13.** A monoclonal antibody having a high degree of specificity and affinity towards one or more compounds of formula (A)

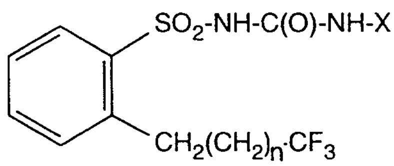

(A),

wherein

X is a mono- or di-substituted 6-membered heterocycle having from one to three nitrogen atoms and bonded via carbon; and
n is an integer from 0 to 4, which monoclonal antibody is obtainable by a method comprising

(a) conjugating a linking component that consists of a fragment that comprises especially the sulfonamide moiety of the compound of formula (A) with a suitable high molecular weight carrier molecule;
(b) immunising a donor animal with the conjugate prepared in accordance with (a);
(c) isolating immunocompetent B cells from the immunised donor animal;
(d) fusing the said immunocompetent B cells with tumour cells capable of continuous cell division;
(e) isolating the resulting fusion product and after selection cloning the hybridoma cells that produce the desired antibody, and
(f) culturing the said hybridoma cells *in vitro* or *in vivo* to produce monoclonal antibodies.

**14.** A monoclonal antibody according to claim 13 having a high degree of specificity and affinity towards one or more compounds of formula (I)

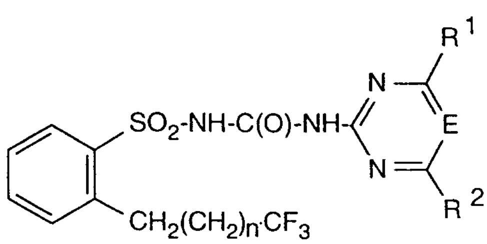

(I),

wherein

$R^1$ and $R^2$ are each independently of the other hydrogen, halogen, $C_1$-$C_4$alkyl, $C_1$-$C_4$haloalkyl, $C_2$-$C_4$haloalkoxy, $C_1$-$C_4$alkylthio, $C_1$-$C_4$haloalkylthio, $C_2$-$C_4$alkoxyalkyl, methoxy, ethoxy or $-NR^3R^4$, wherein
$R^3$ and $R^4$ are each independently of the other hydrogen or $C_1$-$C_4$alkyl;
E is nitrogen or a methine bridge; and
n is an integer from 0 to 4.

**15.** A monoclonal antibody according to claim 14 having a high degree of specificity and affinity towards a compound of formula (II)

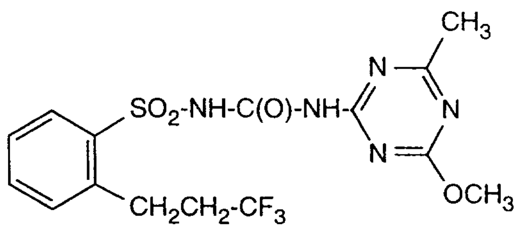

(II).

16. A monoclonal antibody according to any one of claims 13 to 15 wherein in step (a) the said linking component is a compound of formula (III)

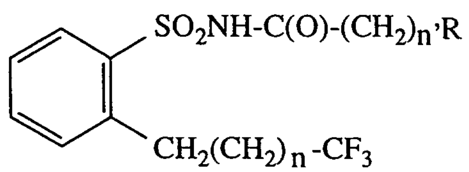

(III),

wherein

R is COOH, $NH_2$ or SH; and
n' is an integer from 1 to 10; and
n is an integer from 0 to 4.

17. Hybridoma cell line ECACC 911 20619, or a clone or subclone thereof.

18. A hybridoma cell line that produces a monoclonal antibody having a high degree of specificity and affinity towards one or more compounds of formula (A)

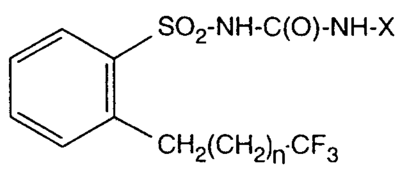

(A),

wherein

X is a mono- or di-substituted 6-membered heterocycle having from one to three nitrogen atoms and bonded via carbon; and
n is an integer from 0 to 4, which monoclonal antibody is obtainable by a method comprising

(a) conjugating a linking component that consists of a fragment that comprises especially the sulfonamide moiety of the compound of formula (A) with a suitable high molecular weight carrier molecule;
(b) immunising a donor animal with the conjugate prepared in accordance with (a);
(c) isolating immunocompetent B cells from the immunised donor animal;
(d) fusing the said immunocompetent B cells with tumour cells capable of continuous cell division; and
(e) isolating the resulting fusion product and after selection cloning the hybridoma cells that produce the desired antibody.

19. A mutant or a variant of the hybridoma cell line according to either claim 17 or claim 18 that occurs spontaneously or that can be produced with the aid of known mutation methods.

20. A method for the immunological detection of sulfonylurea herbicides of the general formula (I), wherein a monoclonal antibody according to either claim 13 or claim 14 is used in one of the known immunoassays.

**21.** A method for the immunological detection of a compound of formula (II), wherein a monoclonal antibody according to either claim 12 or claim 15 is used in one of the known immunoassays.

**22.** A method for the immunological detection of a compound of formula (II) according to claim 21, wherein a monoclonal antibody obtainable from a hybridoma cell line having the distinguishing characteristics of ECACC 911 20619, or from a clone or subclone thereof, is used.

**23.** A method according to any one of claims 20 to 22, wherein the said immunoassay is a competitive immunoassay.

**24.** Composition for the immunological detection of sulfonylurea herbicides of the general formula (I) in the form of a ready-to-use test kit, the said test kit comprising, in addition to the carrier materials, reagents and other additives customarily used, at least one monoclonal antibody according to either claim 13 or claim 14 as reagent.

**25.** Composition for the immunological detection of a compound of formula (II) in the form of a ready-to-use test kit, the said test kit comprising, in addition to the carrier materials, reagents and other additives customarily used, at least one monoclonal antibody according to either claim 12 or claim 15 as reagent.

**26.** Composition for the immunological detection of a compound of formula (II) in the form of a ready-to-use test kit according to claim 25, the said test kit comprising, in addition to the carrier materials, reagents and other additives customarily used, at least one monoclonal antibody that is obtainable from a hybridoma cell line having the distinguishing characteristics of ECACC 911 20619, or from a clone or a subclone thereof.

**27.** A linking component of formula (III)

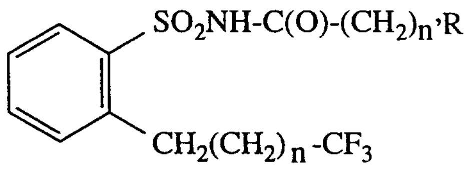

(III)

wherein

R is COOH, $NH_2$ or SH; and
n' is an integer from 1 to 10; and
n is an integer from 0 to 4.

**28.** The use of a monoclonal antibody according to either claim 13 or claim 14 in an immunopurification method, wherein

(a) the said antibody is immobilised on a solid carrier;
(b) the said carrier is placed in a minicolumn;
(c) a buffer solution comprising the sample to be purified or analysed is applied to the column;
(d) optionally one or more washing steps are carried out; and
(e) the analyte is eluted from the column with a suitable elution solution.

**29.** The use of a monoclonal antibody according to either claim 12 or claim 15 in an immunopurification method, wherein

(a) the said antibody is immobilised on a solid carrier;
(b) the said carrier is placed in a minicolumn;
(c) a buffer solution comprising the sample to be purified or analysed is applied to the column;
(d) optionally one or more washing steps are carried out; and
(e) the analyte is eluted from the column with a suitable elution solution.

## Revendications

1. Procédé pour préparer un anticorps monoclonal ayant une grande spécificité et une grande affinité vis-à-vis d'un ou plusieurs composés de formule (A)

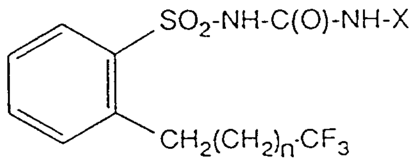

(A)

dans laquelle :

X est un radical hétérocyclique à 6 chaînons, lié par l'intermédiaire d'un carbone, ayant de 1 à 3 atomes d'azote, et
n est un nombre de 0 à 4,
caractérisé en ce que :

(a) on conjugue un composant de couplage, qui est constitue d'un fragment qui en particulier comporte la partie sulfonamide du composé de formule (A), avec une molécule porteuse appropriée à grande masse moléculaire ;
(b) on immunise un animal donneur, avec le conjugué préparé selon (a) ;
(c) à partir de l'animal donneur immunisé, on isole des cellules B immunocompétentes ;
(d) les cellules B immunocompétentes mentionnés ci-dessus sont fusionnées avec des cellules tumorales permettant une division cellulaire continue ;
(e) on isole le produit de fusion ainsi obtenu, et, après sélection, on clone les cellules d'hybridome qui produisent les anticorps souhaités, et
(f) on cultive in vitro ou in vivo les cellules d'hybridome ci-dessus, pour préparer des anticorps monoclonaux.

2. Procédé selon la revendication 1, caractérisé en ce que X est une triazine ou une pyrimidine 1 à 3 fois substituée.

3. Procédé selon la revendication 2 pour préparer un anticorps monoclonal ayant une grande spécificité et une grande affinité vis-à-vis d'un ou plusieurs composés de formule (I)

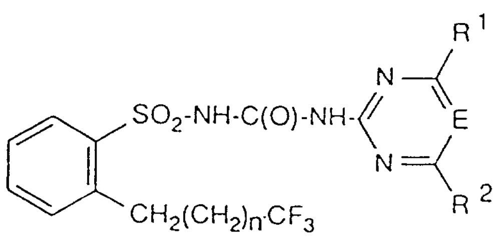

(I)

dans laquelle

$R^1$ et $R^2$, indépendamment de l'autre, sont chacun un hydrogène ou un groupe halogéno, alkyle en $C_1$-$C_4$, halogénalkyle en $C_1$-$C_4$, halogénalcoxy en $C_2$-$C_4$, alkylthio en $C_1$-$C_4$, halogénalkylthio en $C_1$-$C_4$, alcoxyalkyle en $C_2$-$C_4$, méthoxy, éthoxy ou $-NR^3R^4$, dans laquelle
$R^3$ et $R^4$ ,indépendamment l'un de l'autre, sont chacun un hydrogène ou un groupe alkyle en $C_1$-$C_4$ ;
E est l'azote ou le pont méthyne : et
n est un nombre entier de 0 à 4,
caractérisé en ce que :

(a) on conjugue un composant de couplage, qui est constitué d'un fragment qui en particulier comporte la partie sulfonamide du composé de formule (I), avec une molécule porteuse appropriée à grande masse

moléculaire ;

(b) on immunise un animal donneur, avec le conjugué préparé selon (a) ;

(c) à partir de l'animal donneur immunisé, on isole des cellules B immunocompétentes ;

(d) les cellules B immunocompétentes mentionnés ci-dessus sont fusionnées avec des cellules tumorales permettant une division cellulaire continue ;

(e) on isole le produit de fusion ainsi obtenu, et, après sélection, on clone les cellules d'hybridome qui produisent les anticorps souhaités, et

(f) on cultive in vitro ou in vivo les cellules d'hybridome ci-dessus, pour préparer des anticorps monoclonaux.

**4.** Procédé pour préparer un anticorps monoclonal ayant une grande spécificité et une grande affinité vis-à-vis d'un composé de formule (II)

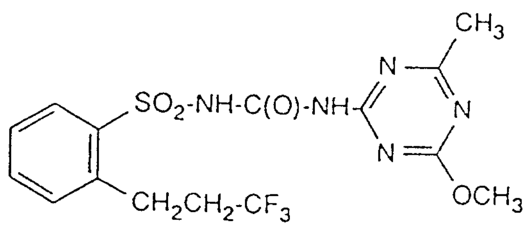

(II)

caractérisé en ce que :

(a) on conjugue un composant de couplage, qui est constitué d'un fragment qui en particulier comporte la partie sulfonamide du composé de formule (II), avec une molécule porteuse appropriée à grande masse moléculaire ;

(b) on immunise un animal donneur, avec le conjugué préparé selon (a) ;

(c) à partir de l'animal donneur immunisé, on isole des cellules B immunocompétentes ;

(d) les cellules B immunocompétentes mentionnés ci-dessus sont fusionnées avec des cellules tumorales permettant une division cellulaire continue ;

(e) on isole le produit de fusion ainsi obtenu, et, après sélection, on clone les cellules d'hybridome qui produisent les anticorps souhaités, et

(f) on cultive in vitro ou in vivo les cellules d'hybridome ci-dessus, pour préparer des anticorps monoclonaux.

**5.** Procédé selon l'une des revendications 1 à 4, caractérisé en ce que, pour ce qui est du composant de couplage mentionné, il s'agit d'un composé de formule (III)

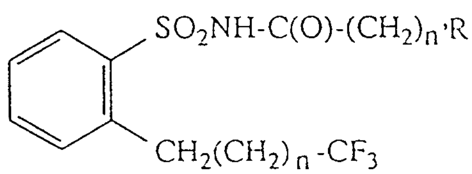

(III)

dans laquelle

R est COOH, $NH_2$ ou SH, et
n' est un nombre entier de 1 à 10, et
n est un nombre entier de 0 à 4.

**6.** Procédé selon l'une des revendications 1 à 4, caractérisé en ce qu'on utilise en tant molécule support des composés macromoléculaires qui comportent des groupes réactifs à accès libre pour la réaction de couplage avec le composant de couplage sulfonylurée, et qui sont à même de conférer au composant sulfonylurée une activité immunogénique.

**7.** Procédé selon l'une des revendications 1 à 4, caractérisé en ce que la conjugaison du composant de couplage sulfonylurée à la molécule support est réalisée directement ou encore par l'intermédiaire d'un chaînon pontant (spacer), qui comporte un ou plusieurs groupes réactifs qui sont à même d'entrer en interaction avec les groupes réactifs de la molécule support.

8. Procédé selon l'une des revendications 1 à 4, caractérisé en ce que l'immunisation des animaux donneurs est réalisée par une à plusieurs administrations d'un conjugué constitué du composant de couplage et de la molécule support.

9. Procédé selon l'une des revendications 1 à 4, caractérisé en ce qu'on utilise le milieu de sélection HAT pour la sélection des cellules hybrides fusionnées.

10. Procédé selon l'une des revendications 1 à 4, caractérisé en ce que, par la procédé des dilutions limites, on individualise les cultures de cellules hybrides positives, produisant des anticorps monoclonaux, puis on clone dans un milieu de culture approprié la culture pure ainsi obtenue.

11. Procédé selon l'une des revendications 1 à 4, caractérisé en ce que l'on détermine la formation d'anticorps monoclonaux appropriés, à l'aide d'une analyse immunologique, dans les clones de cellules d'hybridome clonés selon la revendication 10.

12. Anticorps monoclonal, pouvant être obtenu à partir de la lignée de cellules d'hybridome présentant les éléments caractéristiques de ECACC 911 20 619, et dérivés de cet anticorps monoclonal.

13. Anticorps monoclonal présentant une grande spécificité et une grande affinité vis-à-vis d'un ou plusieurs composés de formule (A)

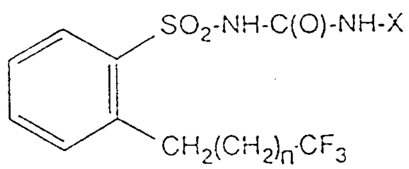

(A)

dans laquelle :

X est un radical hétérocyclique à 6 chaînons, lié par l'intermédiaire d'un carbone, ayant de 1 à 3 atomes d'azote, et
n est un nombre de 0 à 4,
et qui peut être préparé par un procédé caractérisé en ce que :

(a) on conjugue un composant de couplage, qui est constitué d'un fragment qui en particulier comporte la partie sulfonamide du composé de formule (A), avec une molécule porteuse appropriée à grande masse moléculaire ;
(b) on immunise un animal donneur, avec le conjugué préparé selon (a) ;
(c) à partir de l'animal donneur immunisé, on isole des cellules B immunocompétentes ;
(d) les cellules B immunocompétentes mentionnés ci-dessus sont fusionnées avec des cellules tumorales permettant une division cellulaire continue ;
(e) on isole le produit de fusion ainsi obtenu, et, après sélection, on clone les cellules d'hybridome qui produisent les anticorps souhaités, et
(f) on cultive in vitro ou in vivo les cellules d'hybridome ci-dessus, pour préparer des anticorps monoclonaux.

14. Anticorps monoclonal selon la revendication 13, caractérisé en ce qu'il présente une grande spécificité et une grande affinité vis-à-vis d'un ou plusieurs composés de formule (I)

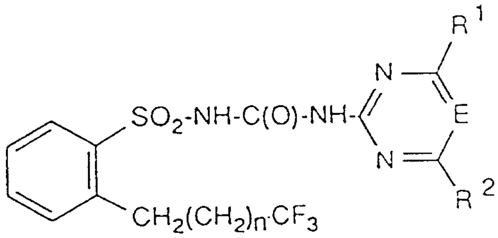

(I)

dans laquelle

$R^1$ et $R^2$, indépendamment de l'autre, sont chacun un hydrogène ou un groupe halogéno, alkyle en $C_1$-$C_4$, halogénalkyle en $C_1$-$C_4$, halogénalcoxy en $C_2$-$C_4$, alkylthio en $C_1$-$C_4$, halogénalkylthio en $C_1$-$C_4$, alcoxyalkyle en $C_2$-$C_4$, méthoxy, éthoxy ou $-NR^3R^4$, dans laquelle
$R^3$ et $R^4$ ,indépendamment l'un de l'autre, sont chacun un hydrogène ou un groupe alkyle en $C_1$-$C_4$ ;
E est l'azote ou le pont méthyne ; et
n est un nombre entier de 0 à 4.

**15.** Anticorps monoclonal selon la revendication 14, caractérisé en ce qu'il présente une grande spécificité et une grande affinité vis-à-vis d'un composé de formule (II)

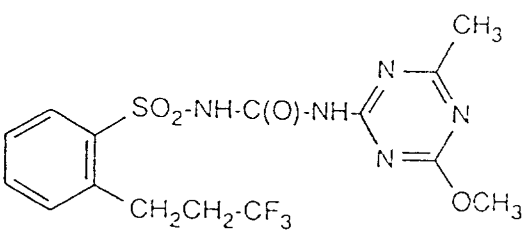

(II)

**16.** Anticorps monoclonal selon l'une des revendications 13 à 15, caractérisé en ce que, dans l'étape (a), et pour ce qui est du composant de couplage mentionné, il s'agit d'un composé de formule (III)

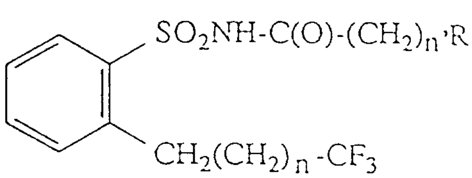

(III)

dans laquelle

R est COOH, $NH_2$ ou SH, et
n' est un nombre entier de 1 à 10, et
n est un nombre entier de 0 à 4.

**17.** Lignée de cellules d'hybridome ECACC 911 20 619, ainsi que ses clones et sous-clones.

**18.** Lignée de cellules d'hybridome produisant un anticorps monoclonal ayant une grande spécificité et une grande affinité vis-à-vis d'un ou plusieurs composés de formule (A)

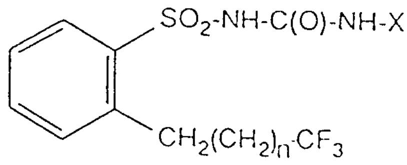

(A)

dans laquelle :

X est un radical hétérocyclique à 6 chaînons, lié par l'intermédiaire d'un carbone, ayant de 1 à 3 atomes d'azote, et
n est un nombre de 0 à 4,
caractérisé en ce que :

(a) on conjugue un composant de couplage, qui est constitué d'un fragment qui en particulier comporte la partie sulfonamide du composé de formule (A), avec une molécule porteuse appropriée à grande masse moléculaire ;
(b) on immunise un animal donneur, avec le conjugué préparé selon (a) ;
(c) à partir de l'animal donneur immunisé, on isole des cellules B immunocompétentes ;
(d) les cellules B immunocompétentes mentionnés ci-dessus sont fusionnées avec des cellules tumorales permettant une division cellulaire continue ;
(e) on isole le produit de fusion ainsi obtenu, et, après sélection, on clone les cellules d'hybridome qui produisent les anticorps souhaités, et
(f) on cultive in vitro ou in vivo les cellules d'hybridome ci-dessus, pour préparer des anticorps monoclonaux.

**19.** Mutants et variants de la lignée de cellules d'hybridome selon l'une des revendications 17 ou 18, qui se produisent d'une manière spontanée ou peuvent être préparés à l'aide de procédés connus de mutation.

**20.** Procédé de détection immunologique d'herbicides à base de sulfonylurée de formule (I), caractérisé en ce qu'on utilise un anticorps monoclonal selon l'une des revendication 13 ou 14 dans l'une des analyses immunologiques connues.

**21.** Procédé de détection immunologique d'un composé de formule (II), caractérisé en ce qu'on utilise un anticorps monoclonal selon l'une des revendications 12 ou 15 dans une analyse immunologique connue.

**22.** Procédé de détection immunologique d'un composé de formule (II) selon la revendication 21, caractérisé en ce qu'on utilise un anticorps monoclonal que l'on peut obtenir à partir d'une lignée de cellules d'hybridome qui présente les éléments caractéristiques de ECACC 911 20 619, ou de clones ou sous-clones de cette dernière.

**23.** Procédé selon l'une des revendications 20 à 22, caractérisé en ce que, pour ce qui est de l'analyse immunologique, il s'agit d'une analyse immunologique compétitive.

**24.** Moyen de détection immunologique d'herbicides à base de sulfonylurée de formule générale (I), sous forme d'un coffret d'essai près à l'emploi, caractérisé en ce que ledit coffret d'essai contient en tant que réactif, outre les matériaux supports, les réactifs et autres additifs couramment utilisés, au moins un anticorps monoclonal selon l'une des revendications 13 ou 14.

**25.** Moyen de détection immunologique d'un composé de formule (II) sous forme d'un coffret d'essai près à l'emploi, caractérisé en ce que ledit coffret d'essais contient en tant que réactif, outre les matériaux supports, réactifs et autres additifs couramment utilisés, au moins un anticorps monoclonal selon l'une des revendications 12 ou 15.

**26.** Moyen de détection immunologique d'un composé de formule (II) sous forme d'un coffret d'essai près à l'emploi selon la revendication 25, caractérisé en ce que ledit coffret d'essai contient, outre les matériaux supports, réactifs et autres additifs couramment utilisés, au moins un anticorps monoclonal que l'on peut obtenir à partir d'une lignée de cellules d'hybridome présentant les éléments caractéristiques de ECACC 911 20 619, ou à partir de clones ou de sous-clones de cette dernière.

**27.** Composant de couplage de formule (III)

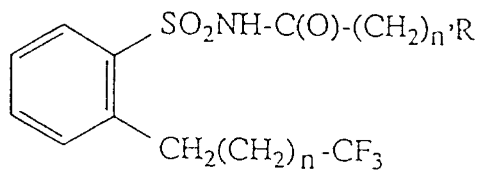

(III)

dans laquelle

R est COOH, $NH_2$ ou SH, et
n' est un nombre entier de 1 à 10, et
n est un nombre entier de 0 à 4.

**28.** Utilisation d'un anticorps monoclonal selon l'une des revendications 13 ou 14 dans un procédé d'immunopurification, caractérisé en ce que :

(a) on immobilise les anticorps ci-dessus sur un support fixe ;
(b) on place les supports dans une mini-colonne ;
(c) on applique sur la colonne une solution tampon contenant l'échantillon à purifier ou à analyser ;
(d) éventuellement, on insère une ou plusieurs étapes de lavage ; et
(e) on élue les analytes de la colonne, avec une solution d'élution appropriée.

**29.** Utilisation d'un anticorps monoclonal selon l'une des revendications 12 ou 15 dans un procédé d'immunopurification, caractérisé en ce que :

(a) on immobilise les anticorps ci-dessus sur un support fixe ;
(b) on place les supports dans une mini-colonne ;
(c) on applique sur la colonne une solution tampon contenant l'échantillon à purifier ou à analyser ;
(d) éventuellement, on insère une ou plusieurs étapes de lavage ; et
(e) on élue les analytes de la colonne, avec une solution d'élution appropriée.